# DEMANDE DE BREVET EUROPEEN

(11) **EP 1 792 605 A1**
(43) Date de publication de la demande: **06.06.2007**
(21) Numéro de dépôt: 06291839.6
(22) Date de dépôt: 30.11.2006
(51) Int. Cl.: A61K 8/41, A61K 8/49, A61Q 5/10

(54) **Utilisation d'au moins un dérivé cationique de cyanine pour la coloration capillaire, composition le contenant, procédé de traitement des fibres kératiniques mettant en oeuvre ladite composition, dispositif et utilisation**

(30) Priorité: 30.11.2005 FR 0512158
(71) Demandeur: L'Oreal, 75008 Paris (FR)
(72) Inventeur: Lagrande, Alain, 77000 Coupvray (FR)
(74) Mandataire: Casalonga, Axel

(57) **Abrégé**

L'invention concerne l'utilisation d'un colorant direct particulier en tant qu'agent colorant et/ou en tant qu'agent d'éclaircissement optique des fibres kératiniques, en particulier humaines.

L'invention porte également sur une composition comprenant, dans un milieu cosmétiquement acceptable, au moins ledit colorant direct particulier.

L'invention concerne de plus un procédé de traitement de fibres kératiniques en particulier humaines, mettant en jeu la composition précitée, ainsi qu'un dispositif la comprenant.

Enfin, elle a pour objet l'utilisation la composition selon l'invention comme agent colorant et/ou comme agent d'éclaircissement optique desdites fibres.

## Description

L'invention concerne l'utilisation d'au moins un colorant direct particulier dérivé de cyanine cationique pour la coloration capillaire. L'invention porte également sur une composition comprenant, dans un milieu cosmétiquement acceptable, au moins un colorant direct particulier dérivé de cyanine cationique. Elle a de même pour objet un procédé de traitement de fibres kératiniques mettant en jeu cette composition, ainsi qu'un dispositif la comprenant. Enfin, elle a pour objet l'utilisation de la composition selon l'invention comme agent colorant et/ou comme agent d'éclaircissement optique desdites fibres.

La présente invention a trait au domaine de la coloration des fibres kératiniques et plus particulièrement de la coloration capillaire.

Il existe essentiellement deux types de coloration.

Le premier est la coloration dite semi-permanente ou coloration directe, qui fait appel à des colorants capables d'apporter à la coloration naturelle des cheveux, une modification plus ou moins marquée. Les colorants mis en jeu sont des substances colorées et colorantes qui présentent une certaine affinité avec la fibre kératinique. Il est à noter que ce type de coloration s'estompe au bout de plusieurs lavages, ce qui peut représenter un inconvénient.

Dans le cas où l'on souhaite obtenir une coloration plus claire que la couleur originale des fibres, il est nécessaire d'utiliser avec les colorants directs, au moins un agent oxydant, dans des conditions de pH alcalin. Cependant, ces conditions de mise en oeuvre ne sont pas sans conséquence sur les propriétés des fibres traitées. En effet, à la longue, les fibres sont plus ou moins dégradées et ont tendance à devenir rêches, ternes, cassantes, difficiles à coiffer.

Le deuxième type de coloration est la coloration permanente ou coloration d'oxydation. Celle-ci est réalisée avec des précurseurs de colorants d'oxydation qui sont des composés incolores ou faiblement colorés, comprenant au moins une base d'oxydation éventuellement associée à un ou plusieurs coupleurs. Une fois mélangés à des produits oxydants, au moment de l'emploi, les précurseurs peuvent donner naissance par un processus de condensation oxydative à des composés colorés et colorants.

Etant donné la présence nécessaire d'un agent oxydant dans ce type de coloration, les inconvénients mentionnés ci-dessus se retrouvent aussi dans ce cas.

Il a été trouvé depuis peu que des compositions comprenant au moins un composé fluorescent présentaient une alternative intéressante aux procédés classiques d'éclaircissement mettant en oeuvre un agent oxydant. Ainsi, pour des cheveux foncés, plus particulièrement dont la hauteur de ton est inférieure ou égale à 6 (blond foncé), de préférence inférieure ou égale à 4 (châtain), on a pu constater qu'il existait des zones pour lesquelles la courbe de réflectance en fonction de la longueur d'onde (entre 500 et 700 nm) des cheveux traités avec la composition comprenant le composé fluorescent, était supérieure à la courbe correspondant aux cheveux non traités. Par conséquent, les cheveux apparaissent éclaircis, sans qu'il soit nécessaire d'utiliser un agent oxydant.

Il est rappelé que la notion de "ton" repose sur la classification des nuances naturelles, un ton séparant chaque nuance de celle qui la suit ou la précède immédiatement. Cette définition et la classification des nuances naturelles sont bien connues des professionnels de la coiffure et publiée dans l'ouvrage « Sciences des traitements capillaires » de Charles ZVIAK 1988, Ed.Masson, pp.215 et 278. Les hauteurs de ton s'échelonnent de 1 (noir) à 10 (blond clair clair), une unité correspondant à un ton ; plus le chiffre est élevé et plus la nuance est claire.

Si de telles compositions constituent une avancée dans ce domaine, il n'en reste pas moins cependant que la stabilité en conservation de ces compositions peut être améliorée.

Par ailleurs il est important de disposer de compositions permettant d'obtenir des colorations suffisamment tenaces vis-à-vis des lavages et de l'action de la lumière ce qui est rarement le cas des colorations obtenues avec les colorants directs qu'ils soient fluorescents ou non fluorescents.

Il a été trouvé de manière totalement inattendue que des colorants directs particuliers dérivés de cyanine cationique permettaient d'obtenir des colorations avec des propriétés de stabilité et de sélectivité (variation de coloration entre les différentes parties d'un cheveu ou d'une chevelure) et de ténacités satisfaisantes En particulier, il a été observé que ces colorants directs particuliers étaient plus stables en milieu oxydant alcalin.

La présente invention a donc pour premier objet l'utilisation d'au moins un composé correspondant à la formule (I) suivante 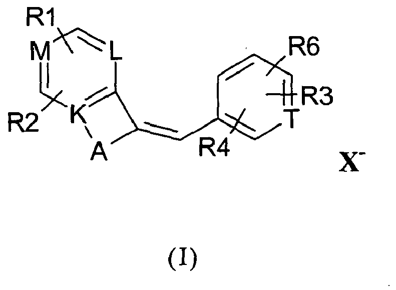 dans laquelle :
M et L représentent indépendamment l'un de l'autre CR₇, CR₉ ou N⁺R₅ ;
K désigne un atome de carbone ou d'azote quaternisé N⁺ ;
T désigne un groupement N⁺R₅, un groupement CR₄ ou un atome d'azote ;
A représente un groupe alkylène CₙH₂ₙ linéaire ou ramifié comportant de 1 à 8 atomes de carbone et éventuellement terminé à l'une de ses extrémités par un atome d'oxygène ou un groupement carbonyle ; un groupe comprenant au moins une insaturation, l'insaturation faisant partie d'un noyau benzénique ou d'un hétérocycle éventuellement substitué par un ou plusieurs radicaux alkyle C₁C₄ ou aryle C₆C_{30 ;}
R₁, R₂, R₃, R₄, R₆, R₇ et R₉, indépendamment les uns des autres, représentent un atome d'hydrogène ; un atome d'halogène ; un groupement aryle en C₆-C₃₀ ; un groupement hydroxy ; un groupement cyano ; un groupement nitro ; un groupement sulfo ; un groupement amino ; un groupement acylamino ; un groupement dialkyl(C₁-C₆)amino ; un groupement dihydroxyalkyl(C₁-C₆)amino ; un groupement alkyl(C₁-C₆)hydroxyalkyl(C₁-C₆)amino ; un groupement alcoxy(C₁-C₆) ; un groupement alcoxy(C₁-C₆)carbonyle ; un groupement carboxyalcoxy en C₁-C₆ ; un groupement pipéridinosulfonyle ; un groupement pyrrolidino ; un groupement alkyle(C₁-C₆)halogénoalkyle(C₁-C₆)amino ; un groupement benzoylalkyle(C₁-C₆) ; un groupement vinyle ; un groupement formyle ; un radical aryle en C₆-C₃₀ éventuellement substitué par un ou plusieurs groupements choisis parmi les groupements hydroxyle, alcoxy en C₁-C₆ linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C₁-C₆ ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C₁-C₆ linéaire, ramifié ou cyclique, , aryle éventuellement substitué, carboxyle, sulfo, un atome d'halogène,ce radical alkyle pouvant être interrompu par un hétéroatome, tel que N, S ou O ;
Deux des substituants R₁, R₂, R₇ et R₉, lorsque M et L représentent respectivement CR₇, CR₉, peuvent former avec les atomes de carbone auxquels ils sont rattachés un cycle aromatique ou non, en C₆-C₃₀ ou hétérocyclique comportant au total de 5 à 30 chaînons et de 1 à 5 hétéroatomes ; ces cycles étant condensés ou non, insérant ou non un groupement carbonyle, et étant substitués ou non par un ou plusieurs groupements choisis parmi les groupements alkyle en C₁-C₄, alcoxy(C₁-C₄)alkyl(C₁-C₄), amino, dialkyl(C₁-C₄)amino, halogène, phényle, carboxy, trialkyl(C₁-C₄)ammonioalkyl(C₁-C₄) ;
R₁, R₂ et R₇ ou R₉ peuvent également représenter un groupe similaire à la partie de la molécule condensée sur le noyau pyridinium de manière à former une molécule parfaitement symétrique, comportant ainsi nécessairement un enchaînement acridinium ;
Deux des substituants R₃, R₄ et R₆ peuvent former avec les atomes de carbone auxquels ils sont rattachés un noyau aromatique en C₆-C₃₀ ou hétérocyclique comportant au total de 5 à 30 chaînons et de 1 à 5 hétéroatomes ; ce cycle étant condensés ou non, ce cycle et l'éventuel cycle condensé étant substitués ou non par au moins un groupement choisi parmi les groupements alkyle en C₁-C₄, alcoxy(C₁-C₄)alkyl(C₁-C₄), amino, dialkyl(C₁-C₄)amino, halogène, phényle, carboxy, trialkyl(C₁-C₄)ammonioalkyl(C₁-C₄), ce cycle peut aussi former avec le troisième substituant et l'atome de carbone le portant un nouveau cycle comportant de 5 à 10 chaînons, saturé ou insaturé ;
R₅ représente un radical alkyle linéaire, ramifié ou cyclique, comprenant de 1 à 22 atomes de carbone, éventuellement substitué par au moins un groupement choisi parmi les groupements hydroxyle, alcoxy en C₁-C₆ linéaire, ramifié ou cyclique, aryle éventuellement substitué, carboxyle, sulfo, un atome d'halogène, ce radical alkyle étant éventuellement interrompu par un hétéroatome tel que N, O ou S ;un radical aryle C₆C₃₀ éventuellement substitué par un ou plusieurs radicaux alkyle C₁C₄, alcoxy C₁C₄, hydroxyle ;
R₅ peut former avec l'atome d'azote le portant et avec un des substituants R₁, R₂, R₇, R₉ et l'atome de carbone portant ce substituant un cycle de 5 à 10 chaînons substitué ou non par un groupement alkyle. Ce cycle peut être condensé éventuellement avec un cycle benzénique ou avec un cycle formé par deux des substituants restants et les atomes de carbone les portant ;
X⁻ représente un anion organique ou minéral ;
un seul de K, L, M désigne nécessairement N⁺R₅ ou N⁺ ;
en tant qu'agent colorant des fibres kératiniques, en particulier humaines, et/ou en tant qu'agent pour l'éclaircissement optique de ces fibres.

Par éclaircissement optique, on entend un éclaircissement visible et visuel obtenu sans l'utilisation d'agent oxydant.

Ces colorants permettent d'obtenir des colorations puissantes, chromatiques, peu sélectives et tenaces.

Le ou les colorants directs selon l'invention permettent également d'obtenir des colorations plus claires que la couleur originale des fibres kératiniques, lorsqu'elle est appliquée sur des fibres foncèes, sans que la présence d'un agent oxydant soit nécessaire. Mais bien entendu, il n'est pas exclu que le ou les colorants soient utilisés en présence d'un tel agent.

Elle a de même pour objet une composition comprenant, dans un milieu cosmétiquement acceptable, d'au moins un colorant direct cationique correspondant à la formule (I) définie ci-dessus.

Elle a également pour objet un procédé de traitement de fibres kératiniques plus particulièrement de fibres kératiniques humaines, dans lequel on applique sur lesdites fibres, sèches ou humides, la composition selon l'invention, pendant un temps suffisant pour développer la coloration, après quoi on rince, on lave éventuellement au shampooing, on rince à nouveau et on sèche ou on laisse sécher les fibres résultantes.

Selon une variante du procédé, on applique sur lesdites fibres, sèches ou humides, la composition selon l'invention, sans rinçage final.

Un autre objet de l'invention porte sur l'utilisation de la composition comme agent colorant ou comme agent d'éclaircissement optique des fibres.

Un autre objet encore de l'invention est constitué par un dispositif comprenant la composition selon l'invention.

Mais d'autres caractéristiques et avantages de l'invention apparaîtront plus clairement à la lecture de la description et des exemples qui vont suivre.

De préférence, le composé de formule (I) est choisi parmi les composés de formules (II) à (V) suivantes : 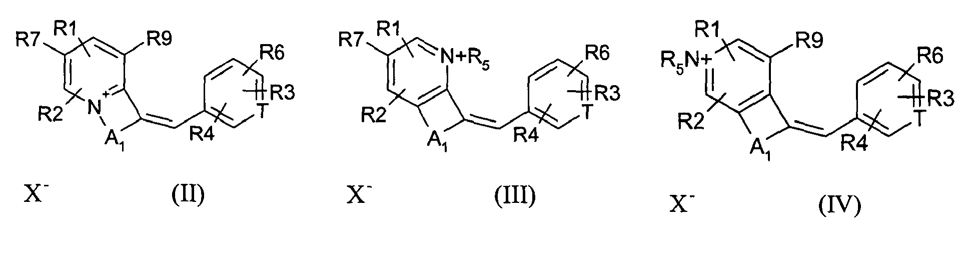 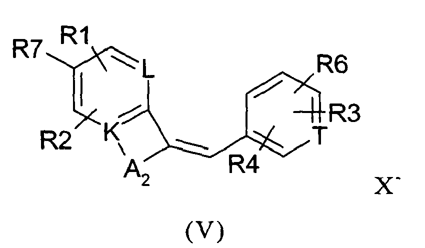 dans lesquelles :
R₁, R₂, R₃, R₄, R₅, R₆, R₇, R₉, K, L, X et T ont les mêmes significations que dans le cas de la formule (I) et dans lesquelles:
   A₁ représente un groupe alkylène CₙH₂ₙ linéaire ou ramifié comportant de 1 à 8 atomes de carbone et éventuellement terminé à l'une de ses extrémités par un atome d'oxygène ou un groupement carbonyle ;
   A₂ représente un groupe comprenant au moins une insaturation, l'insaturation faisant partie d'un noyau benzénique ou d'un hétérocycle éventuellement substitué par un ou plusieurs radicaux alkyle C₁C₄ ou aryle C₆C₃₀ ;
   un seul des groupements K ou L désignant N⁺ ou un groupe N⁺R₅.

Dans ce qui va suivre et à moins qu'une indication différente ne soit indiquée, les bornes d'un domaine de valeurs sont comprises comme faisant partie de ce domaine.

Selon la présente invention, on entend par fibres kératiniques humaines, les cheveux, les cils, et les sourcils.

Selon un mode particulier de réalisation de l'invention, le colorant direct particulier est destiné à être appliqué sur des fibres kératiniques foncées. Plus particulièrement, les fibres kératiniques foncées sont des fibres pigmentées ou bien encore colorées artificiellement, dont la hauteur de ton est inférieure ou égale à 6 et de préférence inférieure ou égale à 4.

Font également partie du cadre de la présente invention, les formes mésomères des composés de formules (I) à (V).

De préférence dans la formule (I) :
R₁, R₂, R₃, R₄, R₆, R₇ et R₉ indépendamment les uns des autres, représentent un atome d'hydrogène ; un groupement aryle en C₆-C₃₀ éventuellement substitué par un groupement choisis parmi les radicaux alkyle en C₁-C₆, alcoxy en C₁-C₆ et hydroxyle ; un groupement hydroxy ; un groupement nitro ; un groupement vinyle ; un groupement pyrrolidino ; un groupement benzoylalkyle(C₁-C₆) ; un groupement alkyl(C₁-C₆)halogénoalkyle(C₁-C₆)amino ; un groupement amino ; un groupement dialkyl(C₁-C₆)amino ; un groupement dihydroxyalkyl(C₁-C₆)amino ; un groupement alkyl(C₁-C₆₎hydroxyalkyl(C₁-C₆)amino ; un groupement alcoxy(C₁-C₆) ; un groupement formyle ; un radical alkyle linéaire, ramifié ou cyclique, comprenant de 1 à 6 atomes de carbone, éventuellement substitué par un ou plusieurs groupements choisis parmi les groupements hydroxyle aryle éventuellement substitué,
R₁, R₂, R₇ et R₉ peuvent former avec les atomes de carbone auxquels ils sont rattachés un cycle aromatique ou non, en C₆-C₃₀ ou hétérocyclique comportant au total de 5 à 30 chaînons et de 1 à 5 hétéroatomes ; ces cycles étant condensés ou non avec insertion ou non d'un groupement carbonyle, et étant substitués ou non par un ou plusieurs groupements choissi parmi les groupements alkyle en C₁-C₄, alcoxy(C₁-C₄)alkyl(C₁-C₄), amino, dialkyl(C₁-C₄)amino, phényle,
R₁, R₂, R₇ et R₉ peuvent également représenter un groupe similaire à la partie de la molécule condensée sur le noyau pyridinium de manière à former une molécule parfaitement symétrique, comportant ainsi nécessairement un enchaînement acridinium ;
R₃ et R₄ peuvent former avec les atomes de carbone auxquels ils sont rattachés un noyau aromatique en C₆-C₃₀ ou hétérocyclique comportant au total de 5 à 30 chaînons et de 1 à 5 hétéroatomes ; ces cycles étant condensés ou non substitués ou non par au moins un groupement alkyle en C₁-C₄ ; ce cycle peut aussi former avec R6 et l'atome de carbone le portant un nouveau cycle comportant de 5 à 10 chaînons, saturé ou insaturé ;
R₅ représente un radical alkyle linéaire, ramifié ou cyclique, comprenant de 1 à 22 atomes de carbone, un radical aryle C₆C₃₀ éventuellement substitué par un ou plusieurs radicaux alkyle C₁C₄, alcoxy C₁C₄, hydroxyle ;
R₅ peut former avec l'atome d'azote le portant et avec R₁ et l'atome de carbone portant ce substituant R₁, un cycle de 5 à 10 chaînons substitué ou non par un groupement alkyle ; ce cycle peut être condensé éventuellement avec un cycle benzénique ou avec un cycle formé par R₁ et R₂ et les atomes de carbone les portant.
De préférence dans la formule (II) :
A₁ représente un groupe alkylène CₙH₂ₙ linéaire ou ramifié comportant de 1 à 8 atomes de carbone et éventuellement terminé à l'une de ses extrémités par un groupement carbonyle ;
R₁, R₂, R₃, R₄, R₆, R₇ et R₉, indépendamment les uns des autres représentent un atome d'hydrogène ; un groupement aryle en C₆-C₃₀ éventuellement substitué par un groupement choisis parmi les radicaux alkyle en C₁-C₆, alcoxy en C₁-C₆ et hydroxyle ; un groupement hydroxy ; un groupement vinyle ; un groupement pyrrolidino ; un groupement benzoylalkyle(C₁-C₆); un groupement alkyl(C₁-C₆)halogénoalkyle(C₁-C₆)amino ; un groupement amino ; un groupement dialkyl(C₁-C₆)amino ; un groupement dihydroxyalkyl(C₁-C₆)amino ; un groupement alkyl(C₁-C₆₎hydroxyalkyl(C₁-C₆)amino ; un groupement alcoxy(C₁-C₆) ; un groupement formyle ; un radical alkyle linéaire, ramifié ou cyclique, comprenant de 1 à 6 atomes de carbone, éventuellement substitué par un ou plusieurs groupement choisis parmi les groupements hydroxyle aryle éventuellement substitué,
R₃ et R₄ peuvent former avec les atomes de carbone auxquels ils sont rattachés un noyau aromatique en C₆-C₃₀ ou hétérocyclique comportant au total de 5 à 30 chaînons et de 1 à 5 hétéroatomes ; ces cycles étant condensés ou non substitués ou non par au moins un groupement alkyle en C₁-C₄ ; ce cycle peut aussi former avec R₆ et l'atome de carbone le portant un nouveau cycle comportant de 5 à 10 chaînons, saturé ou insaturé.
Encore plus préférentiellement :
A₁ représente un groupement alkylène comprenant de 2 à 4 atomes de carbone ;
T désigne CR₄, N⁺R₅ ou N ;
R₁, R₂, R₇ et R₉ désignent un atome d'hydrogène ;
R₃ et R₄ désignent indépendamment l'un de l'autre un atome d'hydrogène ; un radical alkyle en C₁-C₄ (de préférence méthyle) ; un radical alcoxy en C₁-C₄ (de préférence méthoxy), un groupement dialkyl(C₁-C₄)amino (de préférence diméthylamino ou diéthylamino) ; un groupement dihydroxyalkyl(C₁-C₄)amino (de préférence dihydroxyéthylamino); un groupement alkyl(C₁-C₄)hydroxyalkyl(C₁-C₄)amino (de préférence méthylhydroxyéthylamino) ; un groupement pyrrolidino, un groupe formyle ou forment ensemble avec les atomes de carbone les portant un cycle à 5 ou 6 chaînons comportant éventuellement un ou plusieurs hétéroatomes, ce cycle étant éventuellement condensé, et l'ensemble étant éventuellement substitué par un ou plusieurs radicaux alkyle en C₁-C₆ ;
R₆ désigne un atome d'hydrogène ou forme un cycle incluant un chaînon d'un cycle formé par R₃ et/ou R₄ ;
De préférence dans la formule (III) :
A₁ représente un groupe alkylène CₙH₂ₙ linéaire ou ramifié comportant de 1 à 8 atomes de carbone et éventuellement terminé à l'une de ses extrémités par un groupement carbonyle ;
R₁, R₂, R₃, R₄, R₆, et R₇ indépendamment les uns des autres représentent un atome d'hydrogène ; un groupement aryle en C₆-C₃₀ éventuellement substitué par un groupement choisis parmi les radicaux alkyle en C₁-C₆, alcoxy en C₁-C₆ et hydroxyle ; un groupement hydroxy ; un groupement vinyle ; un groupement pyrrolidino ; un groupement benzoylalkyle(C₁-C₆) ; un groupement alkyl(C₁-C₆)halogénoalkyle(C₁-C₆)amino ; un groupement amino ; un groupement dialkyl(C₁-C₆)amino ; un groupement dihydroxyalkyl(C₁-C₆)amino ; un groupement alkyl(C₁-C₆₎hydroxyalkyl(C₁-C₆)amino ; un groupement alcoxy(C₁-C₆) ; un radical alkyle linéaire, ramifié ou cyclique, comprenant de 1 à 6 atomes de carbone, éventuellement substitué par un ou plusieurs groupement choisis parmi les groupements hydroxyle aryle éventuellement substitué ;
R₃ et R₄ peuvent former avec les atomes de carbone auxquels ils sont rattachés un noyau aromatique en C₆-C₃₀ ou hétérocyclique comportant au total de 5 à 30 chaînons et de 1 à 5 hétéroatomes ; ces cycles étant condensés ou non substitués ou non par au moins un groupement alkyle en C₁-C₄. Ce cycle peut aussi former avec R6 et l'atome de carbone le portant un nouveau cycle comportant de 5 à 10 chaînons, saturé ou insaturé ;
R₁ et R₇ peuvent également représenter un groupe similaire à la partie de la molécule condensée sur le noyau pyridinium de manière à former une molécule parfaitement symétrique, comportant ainsi nécessairement un enchaînement acridinium ;
R₅ représente un radical alkyle linéaire, ramifié ou cyclique, comprenant de 1 à 10 atomes de carbone, un radical aryle C₆C₃₀ éventuellement substitué par un ou plusieurs radicaux alkyle C₁C₄, alcoxy C₁C₄, hydroxyle ;
R₅ peut former avec l'atome d'azote le portant et avec R₁ et l'atome de carbone portant ce substituant R₁ un cycle de 5 à 10 chaînons substitué ou non par un groupement alkyle ; ce cycle peut être condensé éventuellement avec un cycle benzénique ou avec un cycle formé par R₁ et R₂ et les atomes de carbone les portant.
Encore plus préférentiellement :
A₁ représente un groupement alkylène comprenant de 2 à 5 atomes de carbone, et éventuellement terminé à l'une de ses extrémités par un groupement carbonyle, et éventuellement substitué par au moins un groupe alkyle en C₁C₆ ;
T désigne CR₄ ;
R₁, R₂ et R₇ indépendamment les uns des autres représentent un atome d'hydrogène, un radical phényle ou forment ensemble avec les atomes de carbone les portant un cycle aromatique en C₆-C₁₂ ou un cycle saturé insérant éventuellement un groupe carbonyle dans ses chaînons, ces cycles étant éventuellement substitués par des groupements alkyle en C₁C₄ (de préférence méthyle) ;
R₅ désigne un radical alkyle en C₁C₄ (de préférence méthyle ou éthyle) ; un radical aryle C₆C₁₂ éventuellement substitué par un radical alkyle en C₁C₄, hydroxy, alcoxy en C₁C₄ (de préférence méthoxy) ; ou forme un cycle incluant un chaînon d'un cycle issu de R₁, R₂ et/ou R₇ et des atomes les portant ;
R₃ et R₆ désignent un atome d'hydrogène ;
R₄ désigne un atome d'hydrogène, un groupement dialkyl(C₁-C₄)amino (de préférence diméthylamino) ou un groupement alkyl(C₁-C₄)halogénoalkyle(C₁-C₄)amino (de préférence éthylchloroéthylamino).
De préférence dans la formule (IV) :
A₁ représente un groupe alkylène CₙH₂ₙ linéaire ou ramifié comportant de 1 à 8 atomes de carbone et éventuellement terminé à l'une de ses extrémités par un groupement carbonyle ;
R₁, R₂, R₃, R₄, R₆ et R₉ indépendamment les uns des autres représentent un atome d'hydrogène ; un groupement aryle en C₆-C₃₀ éventuellement substitué par un groupement choisis parmi les radicaux alkyle en C₁C₆, alcoxy en C₁C₆ et hydroxyle ; un groupement hydroxy ; un groupement vinyle ; un groupement pyrrolidino ; un groupement benzoylalkyle(C₁-C₆) ; un groupement alkyl(C₁-C₆)halogénoalkyle(C1-C6)amino ; un groupement amino ; un groupement dialkyl(C₁-C₆)amino ; un groupement dihydroxyalkyl(C₁-C₆)amino ; un groupement alkyl(C₁-C₆)hydroxyalkyl(C₁-C₆)amino ; un groupement alcoxy(C₁-C₆) ; un radical alkyle linéaire, ramifié ou cyclique, comprenant de 1 à 6 atomes de carbone, éventuellement substitué par un ou plusieurs groupements choisis parmi les groupements hydroxyle aryle éventuellement substitué ;
R₃ et R₄ peuvent former avec les atomes de carbone auxquels ils sont rattachés un noyau aromatique en C₆-C₃₀ ou hétérocyclique comportant au total de 5 à 30 chaînons et de 1 à 5 hétéroatomes ; ces cycles étant condensés ou non substitués ou non par au moins un groupement alkyle en C₁-C₄; ce cycle peut aussi former avec R6 et l'atome de carbone le portant un nouveau cycle comportant de 5 à 10 chaînons, saturé ou insaturé ;
R₅ représente un radical alkyle linéaire, ramifié ou cyclique, comprenant de 1 à 22 atomes de carbone, un radical aryle C₆C₃₀ éventuellement substitué par un ou plusieurs radicaux alkyle C₁C₄, alcoxy C₁C₄, hydroxyle ;
R₅ peut former avec l'atome d'azote le portant et avec R₁ et l'atome de carbone portant ce substituant R₁ un cycle de 5 à 10 chaînons substitué ou non par un groupement alkyle ; ce cycle peut être condensé éventuellement avec un cycle benzénique ou avec un cycle formé par R₁ et R₂ et les atomes de carbone les portant.
Encore plus préférentiellement :
A₁ représente un groupe alkylène CₙH₂ₙ linéaire ou ramifié comportant de 1 à 4 atomes de carbone ;
T désigne CR₄ ;
R₂, R₃ et R₆ représentent un atome d'hydrogène ;
R₁ et R₉ représentent indépendamment l'un de l'autre un atome d'hydrogène, un radical aryle en C₆C₁₂ ou forment ensemble avec les atomes de carbone qui les portent, un cycle aromatique éventuellement condensé(s) en C₆-C₁₂, tous ces cycles pouvant éventuellement être substitués;
R₄ représente un atome d'hydrogène, un groupement dialkyl (C₁-C₆)amino (de préférence diméthylamino, diéthylamino ou dihexylamino), un groupement dihydroxyalkyl(C₁-C₆)amino, un groupement alkyl(C₁-C₆₎hydroxyalkyl(C₁-C₆)amino ;
R₅ représente un groupe alkyle en C₁-C₂₂, phényle, benzyle, ou forme avec R₁ et R₂ et/ou R₉ et les atomes les portant deux cycles accolés.
De préférence dans la formule (V) :
A₂ représente un groupe comprenant au moins une insaturation, l'insaturation faisant partie d'un noyau benzénique ou d'un hétérocycle éventuellement substitué par un ou plusieurs radicaux alkyle C₁C₄ ou aryle C₆C_{30 ;}
R₁, R₂, R₃, R₄, R₆ et R₇ indépendamment les uns des autres représentent un atome d'hydrogène ; un groupement aryle en C₆-C₃₀ éventuellement substitué par un groupement choisis parmi les radicaux alkyle en C₁C₆, alcoxy en C₁C₆ et hydroxyle ; un groupement hydroxy ; un groupement vinyle ; un groupement pyrrolidino ; un groupement benzoylalkyle(C₁-C₆) ; un groupement alkyl(C₁-C₆)halogénoalkyle(C₁-C₆)amino ; un groupement amino ; un groupement dialkyl(C₁-C₆)amino ; un groupement dihydroxyalkyl(C₁-C₆)amino ; un groupement alkyl(C₁-C₆)hydroxyalkyl(C₁-C₆)amino ; un groupement alcoxy(C₁-C₆) ; un groupement formyle ; un radical alkyle linéaire, ramifié ou cyclique, comprenant de 1 à 6 atomes de carbone, éventuellement substitué par un ou plusieurs groupements choisis parmi les groupements hydroxyle aryle éventuellement substitué ;
R₃ et R₄ peuvent former avec les atomes de carbone auxquels ils sont rattachés un noyau aromatique en C₆-C₃₀ ou hétérocyclique comportant au total de 5 à 30 chaînons et de 1 à 5 hétéroatomes ; ces cycles étant condensés ou non substitués ou non par au moins un groupement alkyle en C₁-C₄ ; ce cycle peut aussi former avec R6 et l'atome de carbone le portant un nouveau cycle comportant de 5 à 10 chaînons, saturé ou insaturé ;

Encore plus préférentiellement,
R₁, R₂ et R₇ désignent indépendamment les uns des autres un atome d'hydrogène, un radical alkyle en C₁C₄, vinyle, ou peuvent former ensemble avec les atomes de carbone les portant un cycle saturé ou insaturé portant éventuellement un ou plusieurs radicaux alkyle ;
R₆ représente un atome d'hydrogène ;
R₃ et R₄ représentent indépendamment l'un de l'autre un atome d'hydrogène ; un groupe alkyle C₁-C₄ (de préférence méthyle) ; un groupe dialkylamino (de préférence diméthylamino) ; un groupe alcoxy C₁-C₄ (de préférence méthoxy, éthoxy) ; un radical hydroxy ; un groupe nitro ; un radical benzoylalkyle C₁-C₄ ; ou forment ensemble un cycle à 5 ou 6 chaînons avec les atomes de carbone qui les portent.

Si L représente N⁺R₅, R₅ désigne un radical alkyle C₁-C₄ ou un radical benzoylalkyleC₁-C₄.
Si K représente N⁺, R₉ désigne un atome d'hydrogène.

De préférence, A₂ désigne un enchaînement -C(R₁₀)=C(R₁₁)-, un enchaînement -N=CH-NH- ou un radical pyrazolyle ;
R₁₀, R₁₁ désignant indépendamment l'un de l'autre un radical alkyle en C₁-C₄ (de préférence méthyle), un radical phényle ou forment ensemble un noyau benzénique.

Le colorant direct entrant selon l'invention est généralement une molécule fluorescente, c'est-à-dire qui colore par elle-même, absorbe la lumière du spectre visible et en outre éventuellement de l'ultraviolet (longueurs d'onde allant de 360 à 760 nanomètres) mais qui, contrairement à un colorant classique, transforme une partie de l'énergie absorbée en lumière fluorescente de plus grande longueur d'onde que celle absorbée, émise dans la partie visible du spectre.

En outre, le colorant fluorescent selon l'invention est un colorant qui engendre une fluorescence sur le support sur lequel il est appliqué.

Selon la présente invention, le colorant direct est de préférence soluble dans le milieu d'une composition à au moins 1 gramme par litre et de préférence à au moins 5 grammes par litre à la température de 25°C.

Il est à noter que X- peut être un anion d'origine minérale choisi notamment parmi les halogénures, les sulfates, les bisulfates, les nitrates, les phosphates, les hydrogénophosphates, les dihydrogénophosphates, les boronates, les carbonates, les bicarbonates.

L'anion X⁻ peut aussi être d'origine organique, et dans ce cas, plus particulièrement choisi parmi ceux provenant de sels d'acides mono- ou polycarboxyliques, sulfoniques, sulfuriques, saturés ou non, aromatiques ou non, éventuellement substitués par au moins un radical hydroxyle, amino, ou atomes d'halogène.

De préférence, X- est choisi parmi le chlorure, l'iodure, le sulfate, le méthosulfate, l'éthosulfate.

Conformément à un mode de réalisation particulièrement avantageux de l'invention, le composé de formule (II) correspond à l'un des composés suivants :

| | |
|---|---|
| 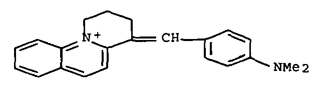 | 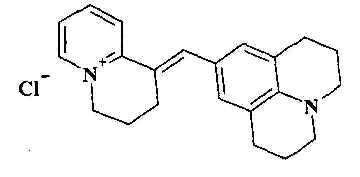 |
| perchlorate de Benzo[c]quinolizinium, 4-[[4-(dimethylamino)phenyl]methylene]-1,2,3,4-tetrahydro-, | Chlorure de 1-(2,3,6,7-Tetrahydro-1H,5H-pyrido[3,2,1-ij]quinolin-9-ylmethylene)-1,2,3,4-tetrahydro-quinolizinylium |
| 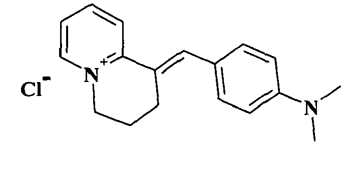 | 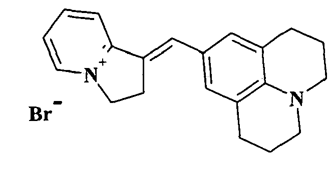 |
| Chlorure de 1-(4-Dimethylaminobenzylidene)-1,2,3,4-tetrahydroquinolizinylium | Bromure de 1-(2,3,6,7-Tetrahydro-1H,5H-pyrido[3,2,1-ij]quinolin-9-ylinethylene)-2,3-dihydro-1H-indolizinylium |
| 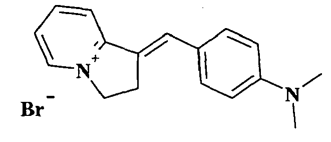 | 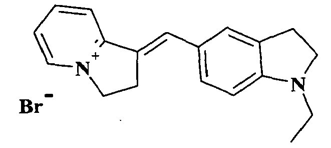 |
| Bromure de 1-(4-Dimethylaminobenzylidene)-2,3-dihydro-1H-indolizinylium | Bromure de 1-(1-Ethyl-2,3-dihydro-1H-indol-5-ylmethylene)-2,3-dihydro-1H-indolizinylium |
| 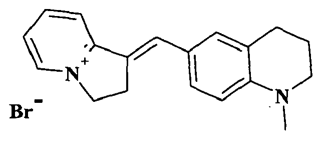 | 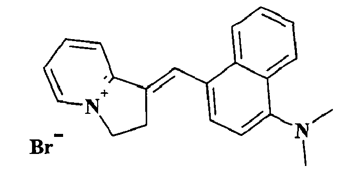 |
| Bromure de 1-(1-Methyl-1,2,3,4-tetrahydro-quinolin-6-ylmethylene)-2,3-dihydro-1H-indolizinylium | Bromure de 1-(4-Dimethylaminonaphthalen-1-ylmethylene)-2,3-dihydro-1H-indolizinylium |
| 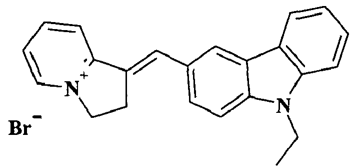 | 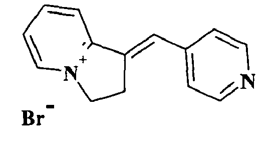 |
| Bromure de 1-(9-Ethyl-9H-carbazol-3-ylmethylene)-2,3-dihydro-1H-indolizinylium | Bromure de 1-Pyridin-4-ylmethylene-2,3-dihydro-1H-indolizinylium |
| 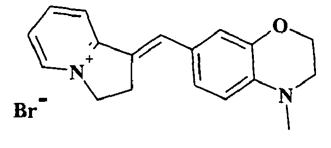 | 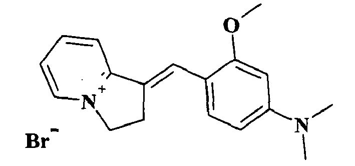 |
| Bromure de 1-(4-Methyl-3,4-dihydro-2H-benzo[1, 4]oxazin-7-ylmethylene)-2,3-dihydro-1H-indolizinylium | Bromure de 1-(4-Dimethylamino-2-methoxy-benzylidene)-2,3-dihydro-1H-indolizinylium |
| 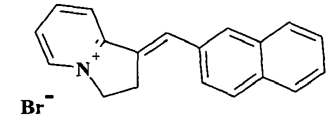 | 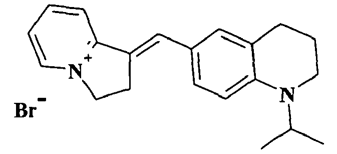 |
| Bromure de 1-Naphthalen-2-ylinethylene-2,3-dihydro-1H-indolizinylium | Bromure de 1-(1-Isopropyl-1,2,3,4-tetrahydro-quinolin-6-ylmethylene)-2,3-dihydro-1H-indolizinylium |
| 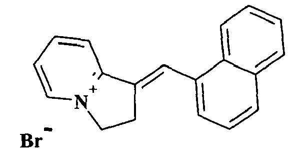 | 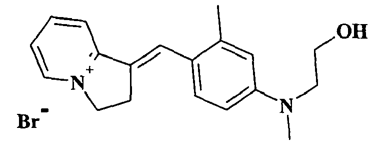 |
| Bromure de 1-Naphthalen-1-ylmethylene-2,3-dihydro-1H-indolizinylium | Bromure de 1-{4-[Ethyl-(2-hydroxy-ethyl)-amino]-2-methyl-benzylidene}-2,3-dihydro-1H-indolizinylium |
| 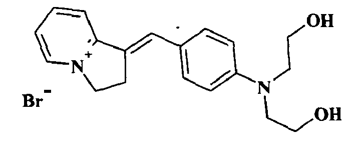 | 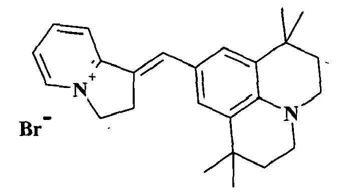 |
| Bromure de 1-{4-[Bis-(2-hydroxy-ethyl)-amino]-benzylidene}-2,3-dihydro-1H-indolizinylium | Bromure de 1-(1,1,7,7-Tetramethyl-2,3,6,7-tetrahydro-1H,5H-pyrido[3,2,1-ij]quinolin-9-ylmethylene)-2,3-dihydro-1H-indolizinylium |
| 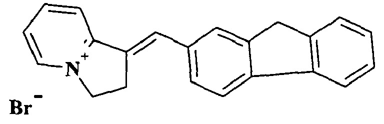 | 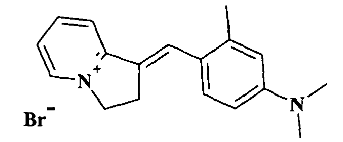 |
| Bromure de 1-(9H-Fluoren-2-ylmethylene)-2,3-dihydro-1H-indolizinylium | Bromure de 1-(4-Dimethylamino-2-methyl-benzylidene)-2,3-dihydro-1H-indolizinylium |
| 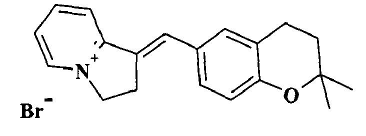 | 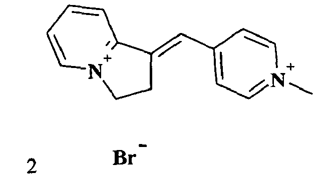 |
| Bromure de 1-(2,2-Dimethyl-chroman-6-ylmethylene)-2,3-dihydro-1H-indolizinylium | Dibromure de 1-Methyl-Pyridimium-4-ylmethylene-2,3-dihydro-1H-indolizinylium |
| 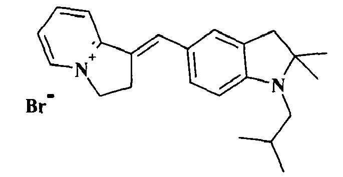 | 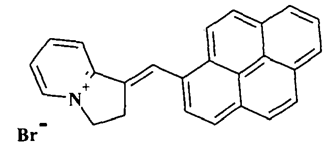 |
| Bromure de 1-(1-Isobutyl-2,2-dimethyl-2,3-dihydro-1H-indol-5-ylmethylene)-2,3-dihydro-1H-indolizinylium | Bromure de 1-Pyren-1-ylmethylene-2,3-dihydro-1H-indolizinylium |
| 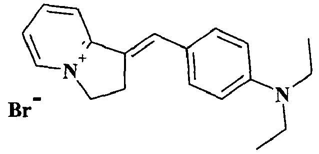 | 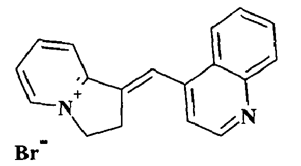 |
| Bromure de 1-(4-Diethylamino-benzylidene)-2,3-dihydro-1H-indolizinylium | Bromure de 1-Quinolin-4-ylmethylene-2,3-dihydro-1H-indolizinylium |
| 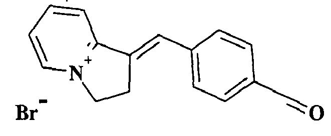 | 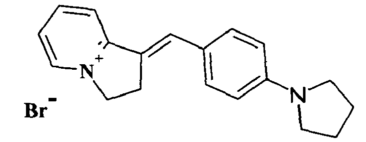 |
| Bromure de 1-(4-Formyl-benzylidene)-2,3-dihydro-1H-indolizinylium; | Bromure de 1-(4-Pyrrolidin-1-yl-benzylidene)-2,3-dihydro-1H-indolizinylium |

Conformément à un mode de réalisation particulièrement avantageux de l'invention, le ccomposé de formule (III) correspond à l'un des composés suivants :

| |
|---|
| 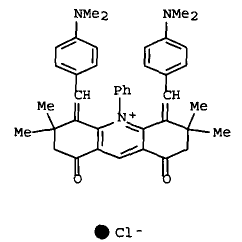 |
| Chlorure de Acridinium, 4,5-bis[[4-(dimethylamino)phenyl]methylene] 1,2,3,4,5,6,7,8-octahydro-3,3,6,6-tetramethyl-1,8-dioxo-10-phenyl |

| | |
|---|---|
| 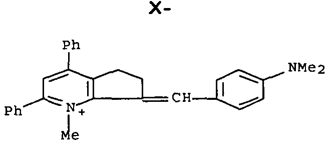 | 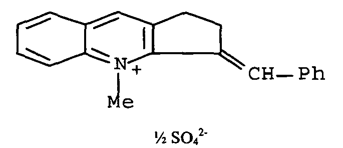 |
| Sel de 5H-Cyclopenta[b]pyridinium, 7-[[4-(dimethylamino)phenyl]methylene]-6,7-dihydro-1-methyl-2,4-diphenyl | Sulfate de 3-Benzylidene-2,3-dihydro-4-methyl-1H-cyclopenta[b]quinolinium methyl |
| 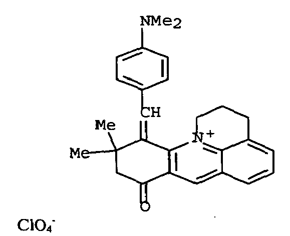 | 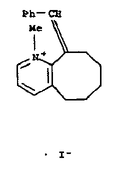 |
| perchlorate de 1H-Pyrido[3,2,1-de]acridinium, 11-[[4-(dimethylamino)phenyl]methylene]-2,3,8,9,10,11-hexahydro-10,10-dimethyl-8-oxo-, | Iodure de Cycloocta[b]pyridinium, 5,6,7,8,9,10-hexahydro-1-methyl-10-(phenylmethylene)-, |
| 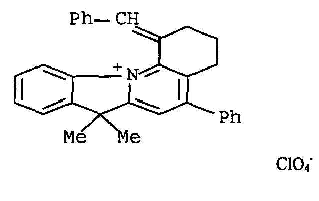 | 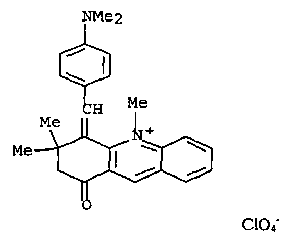 |
| perchlorate de 1H-Indolo[1,2-a]quinolinium, 2,3,4,7-tetrahydro-7,7-dimethyl-5-phenyl-1-(phenylmethylene)-, | perchlorate d'Acridinium, 4-[[4-dimethylamino)phenyl]methylene]-1,2,3,4-tetrahydro-3,3,10-trimethyl-1-oxo |
| 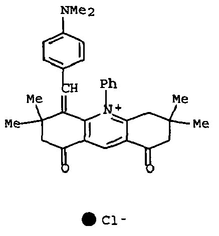 | 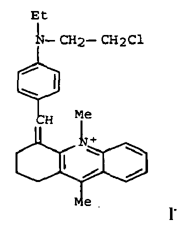 |
| Chlorure d'Acridinium, 4-[[4-(dimethylamino)phenyl]methylene]-1,2,3,4,5,6,7,8-octahydro-3,3,6,6-tetramethyl-1,8-dioxo-10-phenyl | Iodure d'Acridinium, 4-[[4-[(2-chloroethyl)ethylamino]phenyl]methylene]-1,2,3,4-tetrahydro-9,10-dimethyl |
| 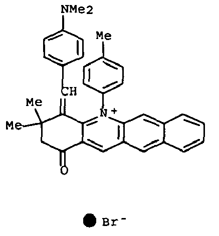 | 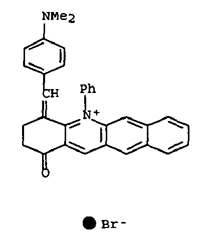 |
| Bromure de Benz[b]acridinium, 4-[[4-(dimethylamino)phenyl]methylene]-1,2,3,4-tetrahydro-3,3-dimethyl-5-(4-methylphenyl)-1-oxo | Bromure de Benz[b]acridinium, 4-[[4-(dimethylamino)phenyl]methylene]-1,2,3,4-tetrahydro-1-oxo-5-phenyl |
| 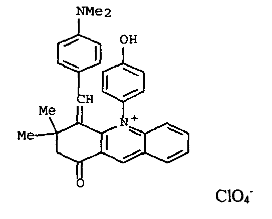 | 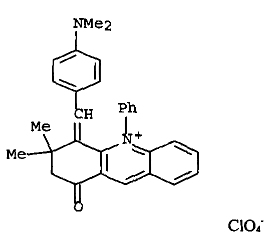 |
| Perchlorate d'Acridinium, 4-[[4-(dimethylamino)phenyl]methylene]-1,2,3,4-tetrahydro-10- (4-hydroxyphenyl)-3,3-dimethyl-1-oxo | Perchlorate d'Acridinium, 4-[[4-(dimethylamino)phenyl]methylene]-1,2,3,4-tetrahydro-3,3-dimethyl-1-oxo-10-phenyl |
| 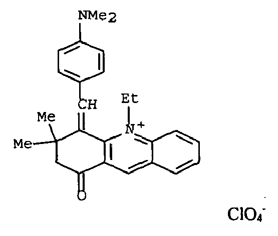 | 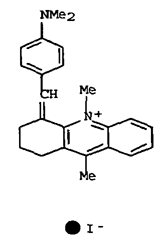 |
| Perchlorate d' Acridinium, 4-[[4-(dimethylamino)phenyl]methylene]-10-ethyl-1,2,3,4-tetrahydro-3,3-dimethyl-1-oxo | Iodure d' Acridinium, 4-[p-(dimethylamino)benzylidene]-1,2,3,4-tetrahydro-9,10-dimethyl |
| 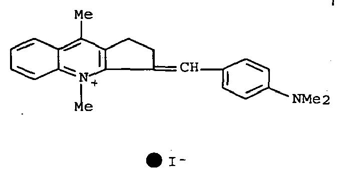 | 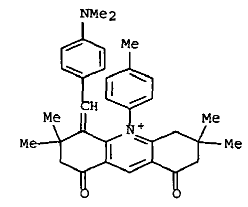 |
| Iodure de 1H-Cyclopenta[b]quinolinium, 3-[p-(dimethylamino)benzylidene]-2,3-dihydro-4,9-dimethyl | Chlorure d'Acridinium, 4-[[4-(dimethylamino)phenyl]methylene]-1,2,3,4,5,6,7,8-octahydro-3,3,6,6-tetramethyl-10-(4-methylphenyl)-1,8-dioxo |
| 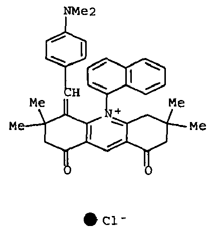 | 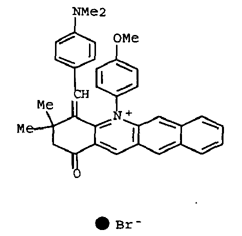 |
| Chlorure d'Acridinium, 4-[[4-(dimethylamino)phenyl]methylene]-1,2,3,4,5,6,7,8-octahydro-3,3,6,6-tetramethyl-10-(1-naphthalenyl)-1,8-dioxo-, | Bromure de Benz[b]acridinium, 4-[[4-(dimethylamino)phenyl]methylene]-1,2,3,4-tetrahydro-5-(4-methoxyphenyl)-3 ,3-dimethyl-1-oxo |
| 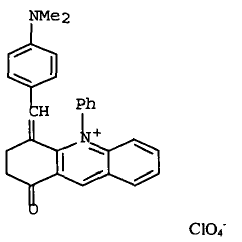 | 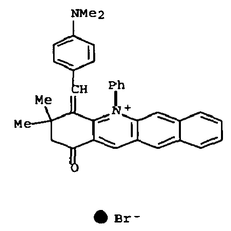 |
| Perchlorate d'Acridinium, 4-[[4-(dimethylamino)phenyl]methylene]-1,2,3,4-tetrahydro-1-oxo-10-phenyl | Bromure de Benz[b]acridinium, 4-[[4-(dimethylamino)phenyl]methylene]-1,2,3,4-tetrahydro-3,3-dimethyl-1-oxo-5-phenyl-, |

Conformément à un mode de réalisation particulièrement avantageux de l'invention, le composé de formule (IV) correspond à l'un des composés suivants :

| | |
|---|---|
| 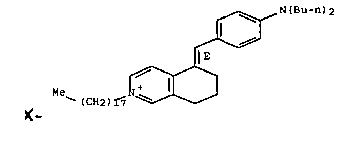 | 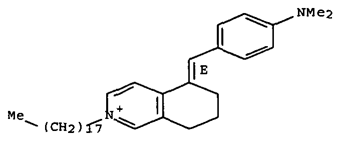 |
| Sel de Isoquinolinium, 5-[[4-(dibutylamino)phenyl]methylene]-5,6,7,8-tetrahydro-2-octadecyl-, (5E) | Sel de Isoquinolinium, 5-[[4-(dimethylamino)phenyl]methylene]-5,6,7,8-tetrahydro-2-octadecyl-, (5E) |
| 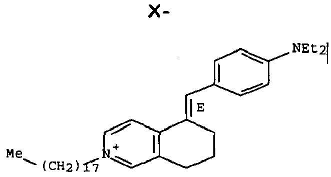 | 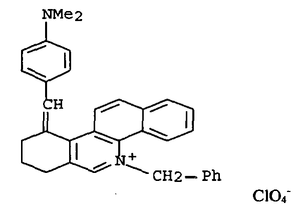 |
| Sel de Isoquinolinium, 5-[[4-(diethylamino)phenyl]methylene]-5,6,7,8-tetrahydro-2-octadecyl-, (5E)-, | Perchlorate de Benzo[c]phenanthridinium, 10-[[4-(dimethylamino)phenyl]methylene]-7,8,9,10-tetrahydro-5-(phenylmethyl)- |
| 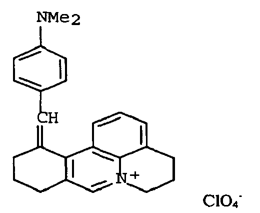 | 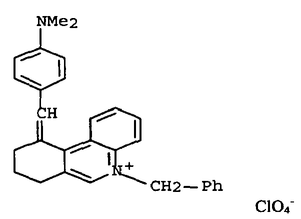 |
| Perchlorate de 4H-Pyrido[3,2,1-de]phenanthridinium, 12-[[4-(dimethylamino)phenyl]methylene]-5,6,9,10,11,12-hexahvdro | Perchlorate de Phenanthridinium, 10-[[4-(dimethylamino)phenyl]methylene]-7,8,9,10-tetrahydro-5-(phenylmethyl |
| 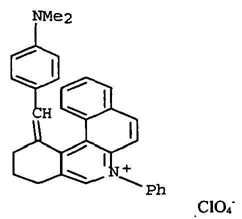 | 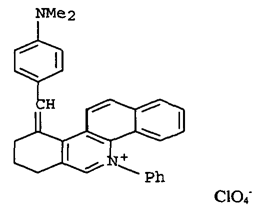 |
| Perchlorate de Benzo[a]phenanthridinium, 1-[[4-(dimethylamino)phenyl]methylene]-1,2,3,4-tetrahydro-6-phenyl-, | Perchlorate de Benzo[c]phenanthridinium, 10-[[4-(dimethylamino)phenyl]methylene]-7,8,9,10-tetrahydro-5-phenyl |
| 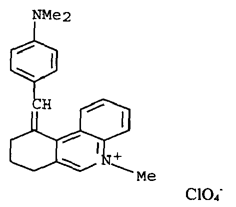 | |
| Perchlorate de Phenanthridinium, 10-[[4-(dimethylamino)phenyl] methylene]-7,8,9,10-tetrahydro-5-methyl | |
| 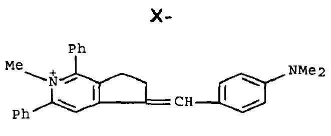 | 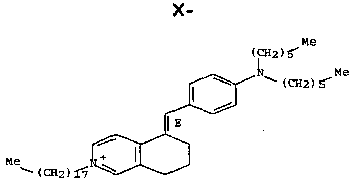 |
| Sel de 5H-Cyclopenta[c]pyridinium, 5-[[4-(dimethylamino)phenyl]methylene]-6,7-dihydro-2-methyl-1,3-diphenyl | Sel de Isoquinolinium, 5-[[4-(dihexylamino)phenyl]methylene]-5,6,7,8-tetrahydro-2-octadecyl-, (5E)-, |
| 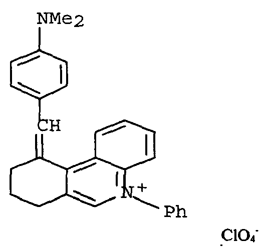 | 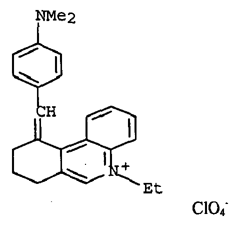 |
| Perchlorate de Phenanthridinium, 10-[[4-(dimethylamino)phenyl] methylene]-7,8,9,10-tetrahydro-5-phenyl | Perchlorate de Phenanthridinium, 10-[[4-(dimethylamino)phenyl]methylene]-5-ethyl-7,8,9,10-tetrahydro |

Conformément à un mode de réalisation particulièrement avantageux de l'invention, le composé de formule (V) correspond à l'un des composés suivants :

| | |
|---|---|
| 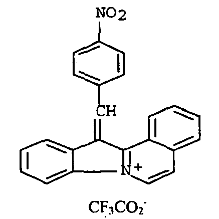 | 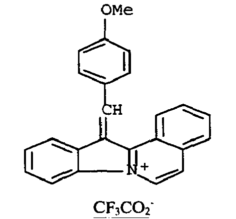 |
| trifluoroacetate de 12H-Indolo[2,1-a]isoquinolinium, 12-[(4-nitrophenyl)methylene]- | trifluoroacetate de 12H-Indolo[2,1-a]isoquinolinium, 12-[(4-methoxyphenyl)methylene]-, |
| 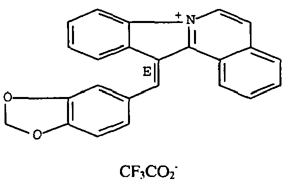 | 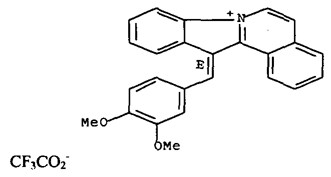 |
| trifluoroacetate de 12H-Indolo[2,1-a]isoquinolinium, 12-(1,3-benzodioxol-5-ylmethylene | trifluoroacetate de 12H-Indolo[2,1-a]isoquinolinium, 12-[(3,4-dimethoxyphenyl)methylene] |
| 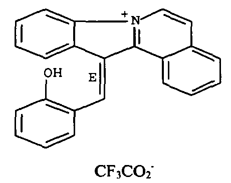 | 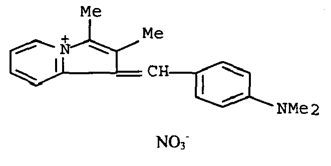 |
| trifluoroacetate de 12H-Indolo[2,1-a]isoquinolinium, 12-[(2-hydroxyphenyl)methylene]- | Nitrate de 1-(p-Dimethylaminobenzylidene)-2,3-dimethyl-1H-indolizinium |
| 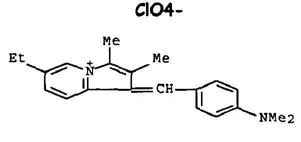 | 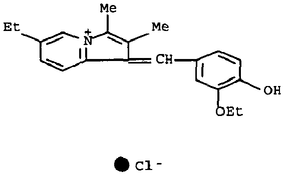 |
| Perchlorate de 1H-Indolizinium, 1-[[4-(dimethylamino)phenyl]methylene]-6-ethyl-2,3-dimethyl- | Chlorure de 1H-Indolizinium, 1-(3-ethoxy-4-hydroxybenzylidene)-6-ethyl-2,3-dimethyl-, |
| 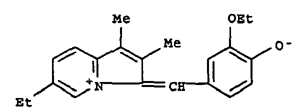 | 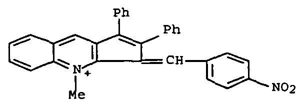 |
| Sel interne de 3H-Indolizinium, 3-(3-ethoxy-4-hydroxybenzylidene)-6-ethyl-1,2-dimethyl-, hydroxide | Perchlorate de 3H-Cyclopenta[b]quinolinium, 4-methyl-3-[(4-nitrophenyl)methylene]-1,2-diphenyl |
| 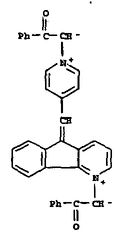 | 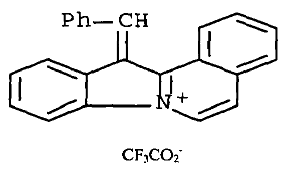 |
| 5H-Indeno[1,2-b]pyridinium, 5-[[1-(2-oxo-2-phenylethyl)pyridinium-4-yl]methylene]-, bis(2-oxo-2-phenylethylide) | trifluoroacetate de 12H-Indolo[2,1-a]isoquinolinium, 12-(phenylmethylene)- |
| 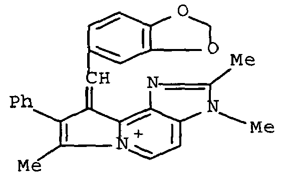 | 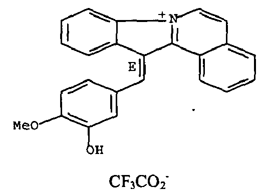 |
| Imidazo[4,5-g]indolizin-6-ium, 9-(1,3-benzodioxol-5-ylmethylene)-3,9-dihydro-2,3,7-trimethyl-8-phenyl | trifluoroacetate de 12H-Indolo[2,1-a]isoquinolinium, 12-[(3-hydroxy-4-methoxyphenyl)methylene]- |
| 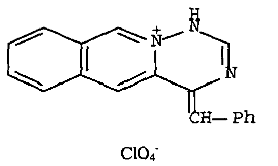 | 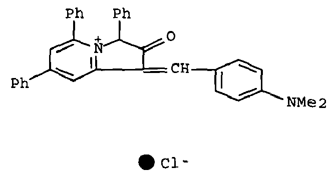 |
| perchlorate de [1,2,4]Triazino[1,6-b]isoquinolin-11-ium, 1,4-dihydro-4-(phenylmethylene)- | Chlorure de 1H-Indolizinium, 1-[[4-(dimethylamino)phenyl]methylene]-2,3-dihydro-2-oxo-3,5,7-triphenyl |
| 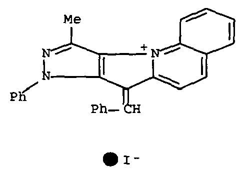 | 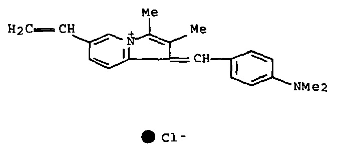 |
| Iodure de Pyrazolo[3',4':4,5]pyrrolo[1,2-a]quinolin-11-ium, 7,8-dihydro-10-methyl-8-phenyl-7-(phenylmethylene)-, | Chlorure de 1H-Indolizinium, 1-[p-(dimethylamino)benzylidene]-2,3-dimethyl-6-vinyl |
| 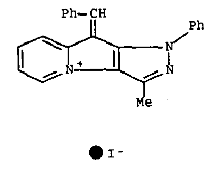 | 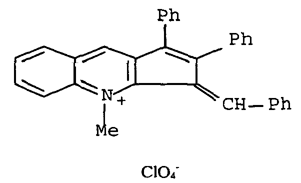 |
| Iodure de Pyrazolo[3,4-b]indolizin-4-ium, 1,9-dihydro-3-methyl-1-phenyl-9- (phenylmethylene)-, | Perchlorate de Cyclopenta[b]quinolinium, 4-methyl-1,2-diphenyl-3-(phenylmethylene) |
| 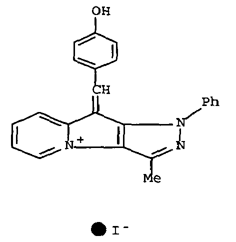 | 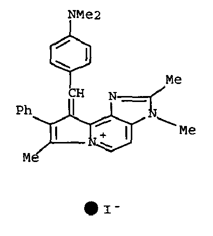 |
| Iodure de Pyrazolo[3,4-b]indolizin-4-ium, 1,9-dihydro-9-[(4-hydroxyphenyl)methylene]- 3-methyl-1-phenyl-, | Iodure de Imidazo[4,5-g]indolizin-6-ium, 9-[[4-(dimethylamino)phenyl]methylene]-3,9-dihydro-2,3,7-trimethyl-8-phenyl |
| 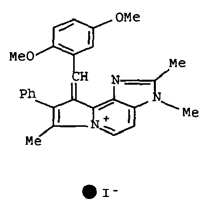 | 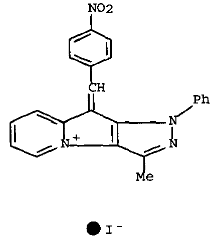 |
| Iodure de Imidazo[4,5-g]indolizin-6-ium, 9-[(2,5-dimethoxyphenyl methylene]-3,9-dihydro-2,3,7-trimethyl-8-phenyl | Pyrazolo[3,4,b]indolizin-4-ium, 1,9-dihydro-3-methyl-9-[(4-nitrophenyl)methylene]1-phenyl |

Les anions mentionnés dans ce tableau ci-dessus ne sont donnés qu'à titre d'exemple.

La synthèse de ces colorants est connue. A titre d'exemple, on peut citer l'article Sczepan Phys.chem.Chem.Phys. 2001,3,3555-3561.

L'invention porte ensuite sur une composition cosmétique comprenant le ou les colorants directs de formules (I) à (V) dans un milieu cosmétiquement acceptable approprié à la teinture des fibres kératiniues, à l'exception des composés suivants :

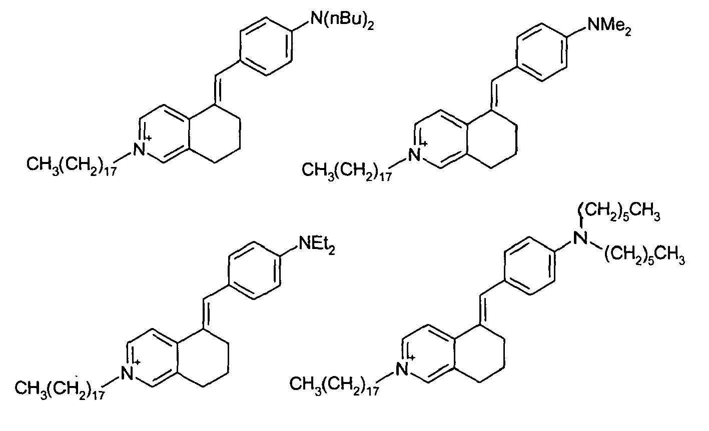

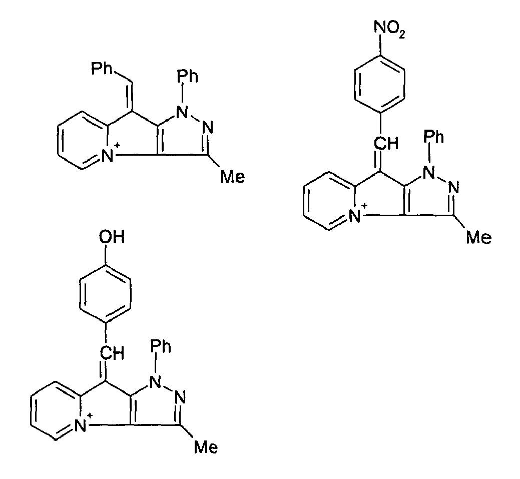

De préférence les compositions de l'uinvention contiennent au moins un agent tensioactif et/ou au moins un polymère épaississant.

Le ou les colorants directs de formules (I) à (V) représentent plus particulièrement de 0,001 à 20 % en poids, de préférence de 0,01 à 5 % en poids par rapport au poids total d'une composition cosmétique.

Le milieu cosmétiquement acceptable est généralement constitué par de l'eau ou par un mélange d'eau et d'au moins un solvant organique.

A titre de solvant organique, on peut par exemple citer, les alcanols, linéaires ou ramifiés, en C₁-C₄, tels que l'éthanol et l'isopropanol ; le glycérol ; les glycols et éthers de glycols comme le 2-butoxyéthanol, le propylèneglycol, le dipropylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Le ou les solvants peuvent être présents dans des proportions allant de préférence de 1 à 40 % en poids environ par rapport au poids total de la composition tinctoriale, et encore plus préférentiellement de 5 à 30 % en poids environ.

Le pH de la composition conforme à l'invention est généralement compris entre 3 et 12 environ, et de préférence entre 5 et 11 environ. Il peut être ajusté à la valeur désirée au moyen d'agents acidifiants ou alcalinisants habituellement utilisés dans le domaine.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VI) suivante : 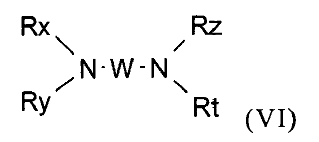 dans laquelle W est un reste propylène éventuellement substitué par un groupement hydroxyle ou un radical alkyle en C₁-C₆ ; Rx, Ry, Rz et Rt, identiques ou différents, représentent un atome d'hydrogène, un radical alkyle en C₁-C₆ ou hydroxyalkyle en C₁-C₆.

La composition cosmétique peut comprendre en outre un ou plusieurs colorants directs additionnels de nature non ionique, cationique ou anionique, et de préférence cationique ou non ionique, ou leurs combinaisons.

Généralement, ces colorants directs sont choisis parmi les colorants benzéniques nitrés, les colorants azoïques, azométhiniques, méthiniques, anthraquinoniques, naphtoquinoniques, benzoquinoniques, phénotiaziniques indigoïdes, xanthéniques, phénanthridiniques, phtalocyanines, ceux dérivés du triarylméthane, les colorants naturels, seuls ou en mélanges.

Il peut par exemple être choisi parmi les colorants benzéniques nitrés rouges ou orangés suivants :
- le 1-hydroxy-3-nitro-4-N-(y-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine,
- lel-amino-3-nitro-6-hydroxybenzène.
- le 1-(β-aminoéthyl)amino-2-nitro-4-(β-hydroxyéthyloxy) benzène,
- le 1-(β, γ-dihydroxypropyl)oxy-3-nitro-4-(β-hydroxyéthyl) amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine,
- le 1-amino-2-nitro-4-hydroxy-5-méthylbenzène.

La composition mise en oeuvre dans le cadre de cette première variante peut également comprendre, en addition ou en remplacement de ces colorants benzéniques nitrés, un ou plusieurs colorants directs choisis parmi les colorants benzéniques nitrés jaunes, jaune-verts, bleus ou violets, les colorants azoïques, les colorants anthraquinoniques, naphtoquinoniques ou benzoquinoniques, les colorants indigoïdes, ou les colorants dérivés du triarylméthane.

Ces colorants directs peuvent notamment être des colorants basiques parmi lesquels on peut citer plus particulièrement les colorants connus dans le Color Index, 3ème édition, sous les dénominations "Basic Brown 16", "Basic Brown 17", "Basic Yellow 57", "Basic Red 76", "Basic Violet 10", "Basic Blue 26" et "Basic Blue 99", ou des colorants directs acides parmi lesquels on peut plus particulièrement citer les colorants connus dans le Color Index, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore des colorants directs cationiques tels que ceux décrits dans les demandes de brevet WO 95/01772, WO 95/15144 et EP 714954 et dont le contenu fait partie intégrante de la présente invention.

Parmi les colorants directs additionnels benzéniques nitrés jaunes et jaune-verts, on peut par exemple citer les composés choisis parmi :
- le 1-β-hydroxyéthyloxy-3-méthylamino-4-nitrobenzène,
- le 1-méthylamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhyl benzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitrobenzène,
- le 1-(β-hydroxyéthyl)amino-2-nitrobenzène,
- le 4-(β-hydroxyéthyl)amino-3-nitrobenzamide.

Parmi les colorants directs additionnels benzéniques nitrés bleus ou violets, on peut par exemple citer les composés choisis parmi :
- le 1-(β-hydroxyéthyl)amino-4-N,N-bis-(β-hydroxyéthyl) amino 2-nitrobenzène,
- le 1-(γ-hydroxypropyl)amino 4-N,N-bis-(β-hydroxy éthyl)amino 2-nitrobenzène,
- le 1-(β-hydroxyéthyl)amino 4-(N-méthyl, N-β-hydroxyéthyl) amino 2-nitrobenzène,
- le 1-(β-hydroxyéthyl)amino 4-(N-éthyl, N-β-hydroxy éthyl)amino 2-nitrobenzène,
- le 1-(β,γ-dihydroxypropyl)amino 4-(N-éthyl, N-β-hydroxy éthyl)amino 2-nitrobenzène,
- les 2-nitroparaphénylènediamines de formule suivante : 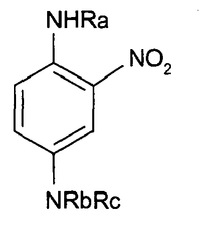
dans laquelle :
Rb représente un radical alkyle en C₁-C₄, un radical β-hydroxyéthyle ou β-hydroxypropyle ou γ-hydroxypropyle ;
Ra et Rc, identiques ou différents, représentent un radical β-hydroxyéthyle, -β-hydroxypropyle, γ-hydroxypropyle, ou β,γ-dihydroxy propyle, l'un au moins des radicaux Rb, Rc ou Ra représentant un radical γ-hydroxypropyle et Rb et Rc ne pouvant désigner simultanément un radical β-hydroxyéthyle lorsque Rb est un radical γ-hydroxypropyle, telles que celles décrits dans le brevet français FR 2 692 572.

Parmi les colorants directs naturels, on peut citer le henné, la camomille, l'indigo, entre autres.

Lorsqu'ils sont présents, le ou les colorants directs additionnels représentent de préférence de 0,0005 à 12 % en poids, et encore plus préférentiellement de 0,005 à 6 % en poids par rapport poids total de la composition.

Lorsqu'elle est destinée à la teinture d'oxydation, la composition cosmétique conforme à l'invention comprend de plus, au moins une base d'oxydation choisie parmi les bases d'oxydation classiquement utilisées pour la teinture d'oxydation et parmi lesquelles on peut notamment citer les paraphénylènediamines, les bis-phénylalkylènediamines, les para-aminophénols, les ortho-aminophénols et les bases hétérocycliques et leurs sels d'addition avec un acide ou avec un agent alcalin.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylène diamine, la N,N-diméthyl para-phénylènediamine, la N,N-diéthyl paraphénylènediamine, la N,N-dipropyl paraphénylène-diamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxy-propyl) paraphénylènediamine, la 2-hydroxy-méthyl paraphénylènediamine, la N,N-diméthyl 3-méthyl paraphénylènediamine, la N,N-(éthyl, β-hydroxyéthyl) paraphénylène diamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylène diamine, la N-(β-méthoxyéthyl) paraphénylène-diamine et la 4'aminophényl 1-(3hydroxy)pyrrolidine, et leurs sels d'addition avec un acide ou avec un agent alcalin.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylène diamine, la 2,3-diméthyl paraphénylènediamine, la N,N-bis-(β-hydroxyéthyl) paraphénylènediamine, la 2-chloro paraphénylène diamine, la 2-β-acétylaminoéthyloxy paraphénylène diamine, et leurs sels d'addition avec un acide ou avec un agent alcalin.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylène diamine, la N,N'-bis-(éthyl) N,N'-bis-(4'-amino, 3'-méthylphényl) éthylène-diamine, le 1,8-bis-(2,5-diamino phénoxy)-3,5-dioxaoctane, et leurs sels d'addition avec un acide ou avec un agent alcalin.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide ou avec un agent alcalin.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide ou avec un agent alcalin.

Parmi les bases hétérocycliques, on peut plus particulièrement citer à titre d'exemple, les dérivés pyridiniques, les dérivés pyrimidiniques et les dérivés pyrazoliques et leurs sels d'addition avec un acide ou avec un agent alcalin.

Lorsqu'elles sont utilisées, la ou les bases d'oxydation représentent de préférence de 0,0005 à 12 % en poids, et encore plus préférentiellement de 0,005 à 6 % en poids, par rapport poids total de la composition.

Lorsqu'elle est destinée à la teinture d'oxydation, la composition selon l'invention peut également comprendre au moins un coupleur de façon à modifier ou à enrichir en reflets les nuances obtenues en mettant en oeuvre les colorants directs et la ou les bases d'oxydation.

Les coupleurs utilisables peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques et leurs sels d'addition avec un acide ou avec un agent alcalin.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, et leurs sels d'addition avec un acide ou avec un agent alcalin.

Lorsqu'ils sont présents, le ou les coupleurs représentent de préférence de 0,0001 à 10 % en poids, et encore plus préférentiellement de 0,005 à 5 % en poids par rapport poids total de la composition.

D'une manière générale, les sels d'addition avec un acide utilisables dans le cadre des compositions de l'invention (bases d'oxydation et coupleurs) sont notamment choisis parmi les chlorhydrates, les bromhydrates, les sulfates, les citrates, les succinates, les tartrates, les tosylates, les benzènesulfonates, les lactates et les acétates.

Les sels d'addition avec un agent alcalin utilisables dans le cadre des compositions de l'invention (bases d'oxydation et coupleurs) sont notamment choisis parmi les sels d'addition avec les métaux alcalins ou alcalino-terreux, avec l'ammoniaque, avec les amines organiques dont les alcanolamines et les composés de formule (VI).

La composition selon l'invention permet d'obtenir des colorations plus claires que la couleur originale des fibres kératiniques, lorsqu'elle est appliquée sur des fibres foncées, sans que la présence d'un agent oxydant soit nécessaire. Mais bien entendu, il n'est pas exclu que la composition selon l'invention comprenne un tel agent.

La composition cosmétique conforme à l'invention peut également comprendre divers adjuvants utilisés classiquement dans les compositions cosmétiques notamment de teinture des fibres kératiniques humaines, tels que des polymères anioniques, cationiques, non ioniques, amphotères, zwittérioniques ou leurs mélanges ; des agents épaississants minéraux ; des agents antioxydants ; des agents de pénétration ; des agents séquestrants ; des parfums ; des tampons ; des agents dispersants ; des agents de conditionnement tels que par exemple des cations, des polymères cationiques ou amphotères, les chitosanes, les silicones volatiles ou non volatiles, modifiées ou non modifiées ; des agents filmogènes ; des céramides ; des agents conservateurs ; des agents stabilisants ; des agents opacifiants.

La composition peut comprendre un ou plusieurs agents tensioactifs. Ces derniers peuvent être indifféremment choisis, seuls ou en mélanges, parmi des tensioactifs anioniques, amphotères, non ioniques, zwittérioniques et cationiques.

Les tensioactifs convenant à la mise en oeuvre de la présente invention sont notamment les suivants :

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C6-C24) sulfosuccinates, les alkyl(C6-C24) éthersulfosuccinates, les alkyl(C6-C24) amidesulfosuccinates ; les alkyl(C6-C24) sulfoacétates ; les acyl(C6-C24) sarcosinates et les acyl(C6-C24) glutamates. On peut également utiliser les esters d'alkyl(C6-C24)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C6-C24) éther carboxyliques polyoxyalkylénés, les acides alkyl(C6-C24)aryl éther carboxyliques polyoxyalkylénés, les acides alkyl(C6-C24) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C10-C14) amines ou les oxydes de N-acylaminopropylmorpholine.

### (iii) Tensioactif(s) amphotère(s) ou zwittérionique(s) :

Les agents tensioactifs amphotères ou zwitterioniques peuvent être notamment choisis parmi les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C8-C20) bétaïnes, les sulfobétaïnes, les alkyl (C8-C20) amidoalkyl (C1-C6) betaïnes ou les alkyl (C8-C20) amidoalkyl (C1-C6) sulfobétaïnes.

Parmi les dérivés d'amines, on peut citer les produits vendus sous la dénomination MIRANOL, tels que décrits dans les brevets US 2528378 et US 2781354 et classés dans le dictionnaire CTFA, 3ème édition, 1982, sous les dénominations Amphocarboxyglycinates et Amphocarboxypropionates de structures respectives :

Rd -CONHCH2CH2 -N(Re)(Rf)(CH2COO-)

dans laquelle : Rd désigne un radical alkyle d'un acide Rd-COOH présent dans l'huile de coprah hydrolysée, un radical heptyle, nonyle ou undécyle, Re désigne un groupement bêta-hydroxyéthyle et Rf un groupement carboxyméthyle ;
et

Rg-CONHCH2CH2-N(B)(C)

dans laquelle :
B représente -CH2CH2OX, C représente -(CH2)z -Y, avec z = 1 ou 2,
X désigne le groupement -CH2CH2-COOH ou un atome d'hydrogène
Y désigne -COOH ou le radical -CH2 - CHOH - SO3H
Rg désigne un radical alkyle d'un acide Rh -COOH présent dans l'huile de coprah ou dans l'huile de lin hydrolysée, un radical saturé ou comprenant une ou plusieurs insaturations, notamment en C7 à C17, plus particulièrement un radical alkyle en C9, C11, C13, C17 ou sa forme iso, un radical C17 insaturé.

Ces composés sont classés dans le dictionnaire CTFA, 5ème édition, 1993, sous les dénominations Disodium Coco-ampho-diacetate, Disodium Lauro-ampho-diacetate, Disodium Capryl-ampho-diacetate, Disodium Caprylo-ampho-diacetate, Disodium Coco-ampho-dipropionate, Disodium Lauro-ampho-dipropionate, Disodium Capryl-ampho-dipropionate, Disodium Caprylo-ampho-dipropionate, Lauro-ampho-dipropionic acid, Coco-ampho-dipropionic acid.

A titre d'exemple on peut citer le cocoamphodiacetate commercialisé sous la dénomination commerciale Miranol® C2M concentré par la société Rhodia Chimie.

### (iv) Tensioactifs cationiques :

Parmi les tensioactifs cationiques on peut citer en particulier les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

De préférence, les tensioactifs sont non ioniques, anioniques ou amphotères.

Habituellement, les agents tensioactifs sont présents dans une quantité comprise entre 0,01 et 50 % en poids, de préférence entre 0,1 et 25 % en poids par rapport au poids total de la composition.

La composition peut de plus comprendre un ou plusieurs polymères épaississants. Ces polymères peuvent être ioniques ou non, associatifs ou non.

En ce qui concerne les polymères épaississants non associatifs, il est tout d'abord rappelé qu'au sens de la présente invention, les polymères épaississants non associatifs sont des polymères épaississants ne contenant pas de chaîne grasse en C₁₀-C₃₀.

Parmi les polymères épaississants non associatifs présents, on peut citer les homopolymères d'acide acrylique réticulés, homopolymères réticulés d'acide 2-acrylamido-2-méthyl-propane sulfonique et leurs copolymères réticulés d'acrylamide, les homopolymères d'acrylate d'ammonium ou les copolymères d'acrylate d'ammonium et d'acrylamide, les gommes de guar non ioniques, les gommes de biopolysaccharides d'origine microbienne, les gommes issues d'exudats végétaux, les hydroxypropyl- ou carboxyméthyl- celluloses ; les pectines et les alginates, seuls ou en mélanges.

Une première famille de polymères épaississants non associatifs convenable est représentée par les homopolymères d'acide acrylique réticulés.

Parmi les homopolymères de ce type, on peut citer ceux réticulés par un éther allylique d'alcool de la série du sucre, comme par exemple les produits vendus sous les noms CARBOPOLS 980, 981, 954, 2984 et 5984 par la société NOVEON ou les produits vendus sous les noms SYNTHALEN M et SYNTHALEN K par la société 3 VSA.

Les polymères épaississants non associatifs peuvent aussi être choisis parmi les homopolymères réticulés d'acide 2-acrylamido-2-méthylpropane sulfonique et leurs copolymères réticulés d'acrylamide.

En ce qui concerne ces homopolymères et copolymères, qui peuvent être partiellement ou totalement neutralisés, on peut citer les polymères comprenant de 90 à 99,9% en poids, par rapport au poids total du polymère, de motifs de formule (j) suivante : 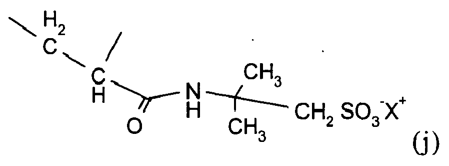 dans laquelle X+ désigne un cation ou un mélange de cations, ou un proton.

Plus particulièrement les cations sont choisis parmi les métaux alcalins (comme le sodium, le potassium), les ions ammonium substitués ou non par un à trois radicaux alkyle, identiques ou différents, comprenant 1 à 6 atomes de carbone, éventuellement porteur d'au moins un radical hydroxyle, les cations dérivant de la N-méthyl-glucamine, d'acides aminés basiques comme l'arginine et la lysine. De préférence, le cation est un ion ammonium ou sodium.

Par ailleurs, le polymère comprend de 0,01 à 10% en poids, par rapport au poids total du polymère; de motifs réticulants provenant d'au moins un monomère ayant au moins deux insaturation éthyléniques (double liaison carbone-carbone).

Les monomères de réticulation ayant au moins deux insaturations éthyléniques sont choisis par exemple parmi l'éther diallylique, le triallylcyanurate, le diallylmaléate, le (méth)acrylate d'allyle, le dipropylèneglycol-diallyléther, les polyglycol-diallyléthers, le triéthylèneglycol-divinyléther, l'hydroquinone-diallyl-éther, le tétrallyl-oxéthanoyle, le di(méth)acrylate de tétra- ou di-éthylèneglycol, la triallylamine, la tétraallyléthylènediamine le triméthylolpropane-diallyléther, le triméthylolpropane triacrylate, le méthylène-bis(méth)acrylamide ou le divinylbenzène, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Pour plus de détail au sujet de ces polymères, on pourra se reporter au document EP 815828.

Parmi les copolymères réticulés d'acide 2-acrylamido-2-méthyl-propane sulfonique et d'acrylamide partiellement ou totalement neutralisés, on peut citer en particulier le produit décrit dans l'exemple 1 du document EP 503 853 et l'on pourra se reporter à ce document pour ce qui a trait à ces polymères.

La composition peut de même comprendre, à titre de polymères épaississants non associatifs, les homopolymères d'acrylate d'ammonium ou les copolymères d'acrylate d'ammonium et d'acrylamide.

A titre d'exemples d'homopolymères d'acrylate d'ammonium, on peut citer le produit vendu sous le nom MICROSAP PAS 5193 par la société HOECHST. Parmi les copolymères d'acrylate d'ammonium et d'acrylamide, on peut citer le produit vendu sous le nom BOZEPOL C NOUVEAU ou le produit PAS 5193 vendus par la société HOECHST. On pourra notamment se référer aux documents FR 2 416 723, US 2798053 et US 2923692 pour ce qui a trait à la description et à la préparation de tels composés.

La composition peut aussi comprendre des homopolymères de diméthylaminoéthyl-méthacrylate quaternisé par le chlorure de méthyle ou les copolymères de diméthylamino-éthylméthacrylate quaternisé par le chlorure de méthyle et d'acrylamide.

Parmi les homopolymères de ce type, on peut citer les produits vendus sous les noms SALCARE 95 et SALCARE 96 par la société CIBA-ALLIED COLLOIDS. Parmi les copolymères de cette famille, on peut citer le produit SALCARE SC92 vendu par CIBA-ALLIED COLLOIDS ou le produit PAS 5194 vendu par HOECHST. Ces polymères sont notamment décrits et préparés dans le document EP 395282 auquel on pourra se référer.

La composition peut aussi comprendre des gommes de guar non ioniques, comme par exemple les gommes de guar non ioniques non modifiées vendues sous la dénomination VIDOGUM GH 175 par la société UNIPECTINE et sous la dénomination JAGUAR C par la société MEYHALL.

Les gommes de guar non ioniques utilisables selon l'invention sont de préférence modifiées par des groupements hydroxyalkyle en C1-C6. Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants, tels que par exemple des oxydes de propylène, avec la gomme de guar, de façon à obtenir une gomme de guar modifiée par des groupements hydroxypropyle.

Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2.

De telles gommes de guar non ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société MEYHALL ou sous la dénomination GALACTASOL 4H4FD2 par la société AQUALON.

A titre de polymères épaississants non associatifs convenables, on peut aussi mentionner les gommes de biopolysaccharides d'origine microbienne telles que les gommes de scléroglucane ou de xanthane.

Conviennent aussi les gommes issues d'exudats végétaux, telles que les gommes arabiques, gommes Ghatti, gommes Karaya et Tragacanthe ; les hydroxypropyl- ou carboxyméthyl- celluloses ; les pectines et les alginates.

Ces polymères sont bien connus de l'homme de l'art et sont notamment décrits dans l'ouvrage de Robert L. DAVIDSON intitulé "Handbook of Water soluble gums and resins" édité chez Mc Graw Hill Book Company (1980).

Parmi les agents épaississants, on préfère plus particulièrement utiliser les systèmes épaississants à base de polymères associatifs bien connus de l'homme de l'art et notamment de nature non ionique, anionique, cationique ou amphotère.

Il est rappelé que les polymères associatifs sont des polymères hydrophiles capables, dans un milieu aqueux, de s'associer réversiblement entre eux ou avec d'autres molécules. Leur structure chimique comprend plus particulièrement au moins une zone hydrophile et au moins une zone hydrophobe. Par groupement hydrophobe, on entend un radical ou polymère à chaîne hydrocarbonée, saturée ou non, linéaire ou ramifiée, comprenant au moins 10 atomes de carbone, de préférence de 10 à 30 atomes de carbone, en particulier de 12 à 30 atomes de carbone et plus préférentiellement de 18 à 30 atomes de carbone. Préférentiellement, le groupement hydrocarboné provient d'un composé monofonctionnel. A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

La composition peut donc comprendre au moins un polymère associatif choisi parmi les polyuréthanes associatifs, plus particulièrement cationiques ou non ioniques, les dérivés de cellulose associatifs, plus particulièrement cationiques ou non ioniques, les vinyllactames associatifs, les polyacides insaturés associatifs, les aminoplaste-éthers associatifs, les polymères ou copolymères associatifs comprenant au moins un monomère à insaturation éthylénique à groupement sulfonique, seuls ou en mélanges.

Parmi les polymères épaississants associatifs, on peut citer les dérivés de polyuréthanes associatifs, comme ceux obtenus par polymérisation :
- environ 20% à 70% en poids d'un acide carboxylique à insaturation α,β-monoéthylénique,
- environ 20 à 80% en poids d'un monomère à insaturation α,β-monoéthylénique non-tensioactif différent du précédent,
- environ 0,5 à 60% en poids d'un mono-uréthane non-ionique qui est le produit de réaction d'un tensioactif monohydroxylé avec un monoisocyanate à insaturation monoéthylénique.

De tels sont notamment décrits dans EP- 173109 et plus particulièrement dans l'exemple 3. Plus précisément, ce polymère est un terpolymère acide méthacrylique / acrylate de méthyle / diméthyl métaisopropényl benzyl isocyanate d'alcool béhényle éthoxylé (40OE) en dispersion aqueuse à 25%.Ce produit est proposé sous la référence VISCOPHOBE DB1000 par la Société AMERCHOL.

Conviennent aussi les polyuréthanes associatifs cationiques dont la famille a été décrite dans la demande FR 0009609. Elle peut être représentée plus particulièrement par la formule générale (A) suivante :

R-X-(P)n-[L-(Y)m]r-L'-(P')p-X'-R' (A)

dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25 ;
n, m, et p valent chacun indépendamment des autres entre 0 et 1000 ;
la molécule contenant au moins une fonction amine protonée ou quaternisée et au moins un groupement hydrophobe.

Dans un mode de réalisation très avantageux, les seuls groupements hydrophobes de ces polyuréthanes sont les groupes R et R' situés aux extrémités de chaîne.

Selon un premier mode de réalisation préféré, le polyuréthane associatif correspond à la formule (A) dans laquelle R et R' représentent tous les deux indépendamment un groupement hydrophobe ; X, X' représentent chacun un groupe L" ; n et p valent entre 1 et 1000 et L, L', L", P, P', Y et m ont la signification indiquée que dans la formule (A).

Selon un autre mode de réalisation préféré de l'invention, le polyuréthane associatif correspond à la formule (A) dans laquelle R et R' représentent tous les deux indépendamment un groupement hydrophobe, X, X' représentent chacun un groupe L", n et p valent 0, et L, L', L", Y et m ont la signification que dans la formule (A) indiquée auparavant.

Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Conformément à un autre mode de réalisation préféré de l'invention, le polyuréthane associatif correspond à la formule (A) dans laquelle R et R' représentent tous les deux indépendamment un groupement hydrophobe ; X et X' représentent tous les deux indépendamment un groupement comportant une amine quaternaire ; n et p valent zéro, et L, L', Y et m ont la signification indiquée dans la formule (A).

La masse moléculaire moyenne en nombre des polyuréthanes associatifs cationiques est habituellement comprise entre 400 et 500000, en particulier entre 1000 et 400000 et idéalement entre 1000 et 300000 g/mol.

Lorsque X et/ou X' désignent un groupement comportant une amine tertiaire ou quaternaire, X et/ou X' peuvent représenter l'une des formules suivantes : 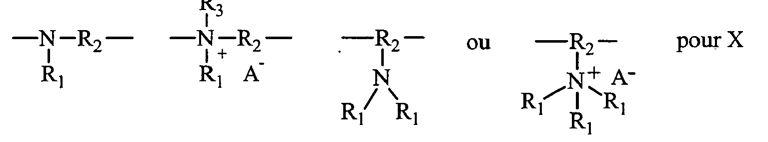 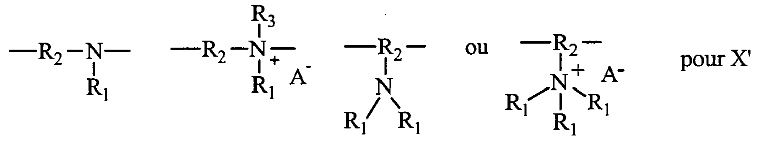 dans lesquelles :
R2 représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R1 et R3, identiques ou différents, désignent un radical alkyle ou alcényle en C1-C30, linéaire ou ramifié, un radical aryle, l'un au moins des atomes de carbone pouvant être remplacé par un hétéroatome choisi parmi N, S, O, P ;
A- est un contre-ion physiologiquement acceptable.

Les groupements L, L' et L" représentent un groupe de formule : 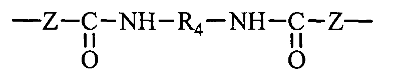 dans laquelle :
Z représente -O-, -S- ou -NH- ; et
R4 représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 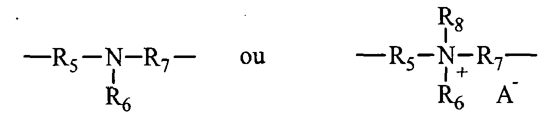 ou 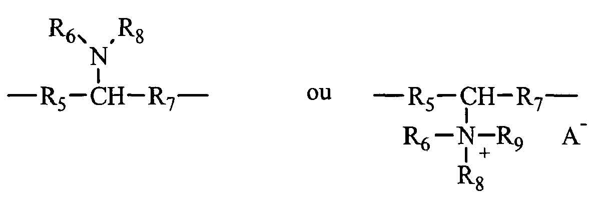 ou 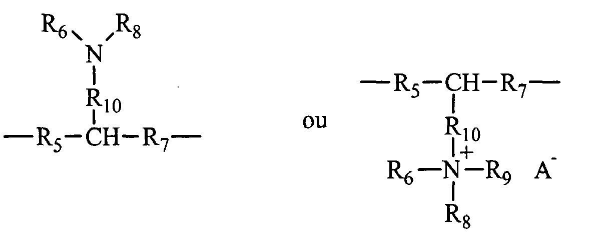 dans lesquelles :
R5 et R7 ont les mêmes significations que R2 défini précédemment ;
R6, R8 et R9 ont les mêmes significations que R1 et R3 définis précédemment ;
R10 représente un groupe alkylène, linéaire ou ramifié, éventuellement insaturé et pouvant contenir un ou plusieurs hétéroatomes choisis parmi N, O, S et P ;
A- est un contre-ion cosmétiquement acceptable.

En ce qui concerne la signification de Y, on entend par groupement hydrophile, un groupement hydrosoluble polymérique ou non. A titre d'exemple, on peut citer, lorsqu'il ne s'agit pas de polymères, l'éthylèneglycol, le diéthylèneglycol et le propylèneglycol. Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs de formule (A) sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" de la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (A) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.

Comme indiqué précédemment, ce composé peut comporter plus d'un motif à fonction amine. Il s'agit alors d'un polymère portant une répétition du motif à fonction amine.

Ce type de composés peut être représenté par l'une des formules suivantes :

HZ-(P)n-ZH, ou HZ-(P')p-ZH

dans lesquelles Z, P, P', n et p sont tels que définis plus haut.

A titre d'exemples de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (A) est un diisocyanate correspondant à la formule O=C=N-R4-N=C=O, dans laquelle R4 est défini plus haut.

On peut citer notamment le méthylènediphényl-diisocyanate, le méthylène cyclohexane diisocyanate, l'isophorone-diisocyanate, le toluènediisocyanate, le naphtalène diisocyanate, le butanediisocyanate, l'hexanediisocyanate.

Un troisième composé entrant dans la préparation du polyuréthane de formule (A) est un composé hydrophobe destiné à former les groupes hydrophobes terminaux du polymère de formule (A).

Ce composé est constitué d'un groupe hydrophobe et d'une fonction à hydrogène labile, par exemple une fonction hydroxyle, amine primaire ou secondaire, ou thiol.

A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné □-hydroxyle.

Le groupe hydrophobe du polyuréthane de formule (A) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.

A titre d'exemples, on peut citer, lorsqu'il ne s'agit pas de polymères, l'éthylèneglycol, le diéthylèneglycol et le propylèneglycol.

Lorsqu'il s'agit d'un polymère hydrophile, on peut citer à titre d'exemple les polyéthers, les polyesters sulfonés, les polyamides sulfonés, ou un mélange de ces polymères.

A titre préférentiel, le composé hydrophile est un polyéther et notamment un poly(oxyde d'éthylène) ou poly(oxyde de propylène).

Le groupe hydrophile noté Y dans la formule (A) est facultatif. En effet, les motifs à fonction amine quaternaire ou protonée peuvent suffire à apporter la solubilité ou l'hydrodispersibilité nécessaire pour ce type de polymère dans une solution aqueuse.

Bien que la présence d'un groupe Y hydrophile soit facultative, on préfère cependant des polyuréthanes associatifs cationiques comportant un tel groupe.

Les dérivés de polyuréthanes associatifs de l'invention peuvent être aussi des polyuréthanes polyéthers non ioniques. Plus particulièrement, lesdits polymères comportent dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, ces polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non ioniques à chaîne grasse peuvent être des copolymères triblocs dont la séquence hydrophile est une chaîne polyoxyéthylénée comportant de 50 à 1000 groupements oxyéthylénés.

Les polyéthers polyuréthanes non ioniques comportent une liaison uréthane entre les séquences hydrophiles, d'où l'origine du nom.

Par extension figurent aussi parmi les polyéthers polyuréthanes non ioniques à chaîne hydrophobe, ceux dont les séquences hydrophiles sont liées aux séquences hydrophobes par d'autres liaisons chimiques.

A titre d'exemples de polyéthers polyuréthanes non ioniques à chaîne hydrophobe utilisables dans l'invention, on peut utiliser aussi le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C12-C14 et le produit ELFACOS T212® à chaîne alkyle en C18 de chez AKZO.

Le produit DW 1206B® de chez ROHM & HAAS à chaîne alkyle en C20 et à liaison uréthane, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemples, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéthers polyuréthanes utilisables décrits auparavant peuvent aussi être choisis parmi ceux décrits dans l'article de G. Fonnum, J. Bakke et Fk. Hansen - Colloid Polym. Sci 271, 380.389 (1993).

Plus particulièrement encore, selon l'invention, on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

La composition peut de même comprendre des polymères dérivés de celluloses associatifs tels que :
- les celluloses cationiques quaternisées modifiées par des groupements comportant au moins une chaîne hydrophobe, tels que les groupes alkyle, arylalkyle, alkylaryle comportant au moins 8 atomes de carbone, ou des mélanges de ceux-ci,
- les hydroxyéthylcelluloses cationiques quaternisées modifiées par des groupements comportant au moins une chaîne hydrophobe, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthyl celluloses quaternisées à chaîne hydrophobe en C8-C30, les produits QUATRISOFT LM 200®, QUATRISOFT LM-X 529-18-A®, QUATRISOFT LM-X 529-18B® (alkyle en C12) et QUATRISOFT LM-X 529-8® (alkyle en C18) commercialisés par la société AMERCHOL et les produits CRODACEL QM®, CRODACEL QL® (alkyle en C12) et CRODACEL QS® (alkyle en C18) commercialisés par la société CRODA.
- les dérivés de cellulose non ioniques tels que les hydroxyéthylcelluloses modifiées par des groupements comportant au moins une chaîne hydrophobe tels que des groupes alkyle, arylalkyle, alkylaryle, ou leurs mélanges, et dans lesquels les groupes alkyle sont de préférence en C8-C22, comme le produit NATROSOL PLUS GRADE 330 CS® (alkyles en C16) vendu par la société AQUALON, ou le produit BERMOCOLL EHM 100® vendu par la société BEROL NOBEL,
- les dérivés de cellulose modifiée par des groupes polyalkylène glycol éther d'alkyl phénol, tel que le produit AMERCELL POLYMER HM-1500® vendu par la société AMERCHOL.

En ce qui concerne les polyvinyllactames associatifs, on peut citer par exemple les polymères notamment décrits dans FR 0101106. Lesdits polymères sont plus particulièrement des polymères cationiques et comprennent :
-a) au moins un monomère de type vinyl lactame ou alkylvinyllactame;
-b) au moins un monomère de structures (a) ou (b) suivantes : 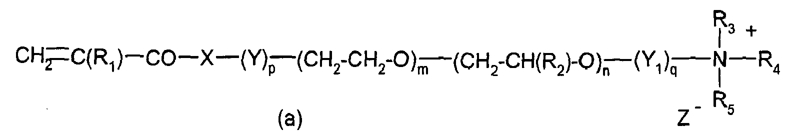 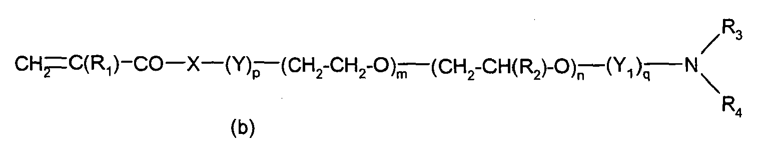 dans lesquelles :
   X désigne un atome d'oxygène ou un radical NR6,
   R1 et R6 désignent, indépendamment l'un de l'autre, un atome d'hydrogène ou un radical alkyl linéaire ou ramifié en C1-C5,
   R2 désigne un radical alkyle linéaire ou ramifié en C1-C4,
   R3, R4 et R5 désignent, indépendamment l'un de l'autre, un atome d'hydrogène, un radical alkyle linéaire ou ramifié en C1-C30 ou un radical de formule (c) :

      -(Y₂)ᵣ-(CH₂-CH(R₇)-O)ₓ-R₈ (c)
   Y, Y1 et Y2 désignent, indépendamment l'un de l'autre, un radical alkylène linéaire ou ramifié en C₂-C₁₆,
   R₇ désigne un atome d'hydrogène, ou un radical alkyle linéaire ou ramifié en C₁-C₄ ou un radical hydroxyalkyle linéaire ou ramifié en C₁-C₄,
   R₈ désigne un atome d'hydrogène ou un radical alkyle linéaire ou ramifié en C₁-C₃₀,
   p, q et r désignent, indépendamment l'un de l'autre, soit la valeur zéro, soit la valeur 1,
   m et n désignent, indépendamment l'un de l'autre, un nombre entier allant de 0 à 100,
   x désigne un nombre entier allant de 1 à 100,
   Z désigne un anion d'acide organique ou minéral,
   sous réserve que :
   - l'un au moins des substituants R₃, R₄, R₅ ou R₈ désigne un radical alkyle linéaire ou ramifié en C₉-C₃₀,
   - si m ou n est différent de zéro, alors q est égal à 1,
   - si m ou n sont égaux à zéro, alors p ou q est égal à 0.

Les polymères poly(vinyllactame) peuvent être réticulés ou non réticulés et peuvent aussi être des polymères blocs.

De préférence le contre ion Z- des monomères de formule (b) est choisi parmi les ions halogénures, les ions phosphates, l'ion méthosulfate, l'ion tosylate.

De préférence R₃, R₄ et R₅ désignent, indépendamment l'un de l'autre, un atome d'hydrogène ou un radical alkyle linéaire ou ramifié en C₁-C₃₀.

Plus préférentiellement, le monomère b) est un monomère de formule (b) pour laquelle, encore plus préférentiellement, m et n sont égaux à zéro.

Le monomère vinyllactame ou alkylvinyllactame est de préférence un composé de structure (d) : 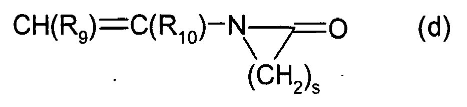 dans laquelle :
s désigne un nombre entier allant de 3 à 6,
R₉ désigne un atome d'hydrogène ou un radical alkyle en C₁-C₅,
R₁₀ désigne un atome d'hydrogène ou un radical alkyle en C₁-C₅,
sous réserve que l'un au moins des radicaux R₉ et R₁₀ désigne un atome d'hydrogène.

Encore plus préférentiellement, le monomère (d) est la vinylpyrrolidone.

Les polymères poly(vinyllactame) peuvent également contenir un ou plusieurs monomères supplémentaires, de préférence cationiques ou non ioniques.

A titre de composés plus particulièrement préférés selon l'invention, on peut citer les terpolymères suivants comprenant au moins :
a)-un monomère de formule (d),
b)-un monomère de formule (a) dans laquelle p=1, q=0, R₃ et R₄ désignent, indépendamment l'un de l'autre, un atome d'hydrogène ou un radical alkyle en C₁-C₅ et R₅ désigne un radical alkyle en C₉-C₂₄ et
c)-un monomère de formule (b) dans laquelle R₃ et R₄ désignent, indépendamment l'un de l'autre, un atome d'hydrogène ou un radical alkyle en C₁-C₅.

Encore plus préférentiellement, on met en oeuvre des terpolymères comprenant, en poids, 40 à 95% de monomère (d), 0,1 à 55% de monomère (b) et 0,25 à 50% de monomère (b).

De tels polymères sont notamment décrits dans la demande de brevet WO 00/68282 dont le contenu fait partie intégrante de l'invention.

Comme polymères poly(vinyllactame), on utilise notamment les terpolymères vinylpyrrolidone / diméthylaminopropylméthacrylamide / tosylate de dodécyl diméthylméthacrylamidopropylammonium, les terpolymères vinylpyrrolidone / diméthyl aminopropylméthacrylamide / tosylate de cocoyldiméthylméthacrylamido propyl ammonium, les terpolymères vinylpyrrolidone / diméthylaminopropyl méthacrylamide / tosylate ou chlorure de lauryldiméthylméthacrylamidopropylammonium. Le terpolymère vinylpyrrolidone / diméthylaminopropyl méthacrylamide /chlorure de lauryl diméthylméthacrylamidopropylammonium est proposé dans l'eau à 20% par la Société ISP sous la dénomination STYLEZE W20.

Les dérivés de polyvinyllactames associatifs de l'invention peuvent être aussi des copolymères non ioniques de vinylpyrrolidone et de monomères hydrophobes à chaîne hydrophobe dont on peut citer à titre d'exemple :
- les produits ANTARON V216® ou GANEX V216® (copolymère vinylpyrrolidone / hexadécène) vendu par la société I.S.P.
- les produits ANTARON V220® ou GANEX V220® (copolymère vinylpyrrolidone / eicosène) vendu par la société I.S.P.

Parmi les dérivés de polyacides insaturés associatifs on peut citer ceux comportant au moins un motif hydrophile de type acide carboxylique insaturé oléfinique, et au moins un motif hydrophobe de type ester d'alkyl (C10-C30) d'acide carboxylique insaturé.

Ces polymères sont notamment choisis parmi ceux dont le motif hydrophile de type acide carboxylique insaturé oléfinique correspond au monomère de formule (e) suivante : 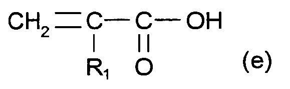 dans laquelle, R1 désigne H ou CH3 ou C2H5, c'est-à-dire des motifs acide acrylique, acide méthacrylique ou acide éthacrylique, et dont le motif hydrophobe de type ester d'alkyl (C10-C30) d'acide carboxylique insaturé correspond au monomère de formule (f) suivante : 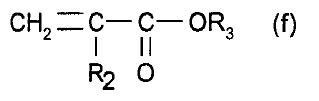 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.

Des esters d'alkyle (C₁₀-C₃₀) d'acides carboxyliques insaturés comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères anioniques de ce type sont par exemple décrits et préparés, selon les brevets US-3 915 921 et 4 509 949.

Dans ce type de polymères associatifs anioniques, on utilise plus particulièrement des polymères formés à partir d'un mélange de monomères comprenant :
essentiellement de l'acide acrylique,
un ester de formule (f) décrite ci-dessus et dans laquelle R₂ désigne H ou CH₃, R₃ désignant un radical alkyle ayant de 12 à 22 atomes de carbone,
et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères associatifs anioniques, on préfère ceux constitués de 95 à 60% en poids d'acide acrylique (motif hydrophile), de 4 à 40% en poids d'acrylate d'alkyles en C10-C30 (motif hydrophobe), et de 0 à 6% en poids de monomère polymérisable réticulant, ou bien ceux constitués de 98 à 96% en poids d'acide acrylique (motif hydrophile), de 1 à 4% en poids d'acrylate d'alkyles en C10-C30 (motif hydrophobe), et de 0,1 à 0,6% en poids de monomère polymérisable réticulant tel que ceux décrits précédemment.

Parmi lesdits polymères ci-dessus, on préfère tout particulièrement les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.

Parmi les dérivés de polyacides insaturés associatifs on peut aussi citer ceux comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C₁-C₄.

A titre d'exemples de ce type de composés, on peut citer l'ACULYN 22® vendu par la société ROHM et HAAS, qui est un terpolymère acide méthacrylique/acrylate d'éthyle/méthacrylate de stéaryle oxyalkyléné.

En ce qui concerne les polymères épaississants du type des aminoplaste-éther, on désigne tout produit issu de la condensation d'un aldéhyde avec une amine ou un amide, ainsi que toute unité structurale formée d'un résidu aminoplaste et d'un résidu hydrocarboné divalent lié au résidu aminoplaste par une liaison éther.

Les polymères à squelette aminoplaste-éther sont choisis de préférence parmi ceux contenant au moins un motif de structure (g) suivante : 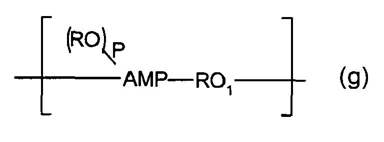 dans laquelle :
- AMP est un résidu aminoplaste avec des unités alkylènes (ou alkyle divalent),
- R désigne un atome d'hydrogène, un radical alkyle C₁-C₄ ou un radical acyle C₁-C₄,
- RO₁ est un résidu alkylène-oxy divalent,
- p désigne un nombre entier positif,
- le ou les groupements OR étant liés aux unités alkylènes du résidu AMP.

De préférence, les polymères à squelette aminoplaste-éther sont choisis parmi ceux contenant au moins un motif de structure (h) suivante : 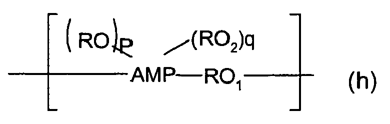 dans laquelle :
- AMP, R, RO1 et p ont la même signification que précédemment,
- RO2 est un groupe différent de RO lié à AMP au moyen d'un hétéroatome et comprenant au moins deux atomes de carbone, et,
- q est un nombre entier positif.

Plus préférentiellement encore, les polymères correspondent aux formules (m) et (n) suivantes : 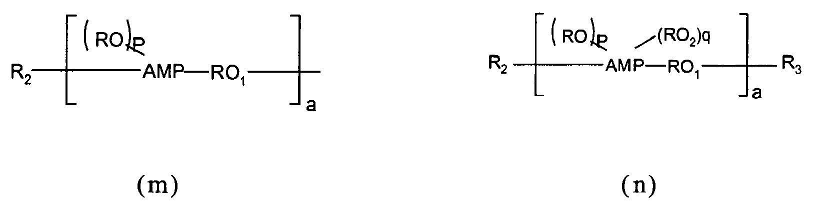 dans lesquelles :
- AMP, R, RO₁, RO₂, p et q ont la même signification que précédemment,
- R₂ ou R₃, identiques ou différents représentent un groupe terminal pouvant désigner un atome d'hydrogène, un groupement RO₁H, un groupement RO₂H, un groupement AMP(OR)p ou tout groupement monofonctionnel tel que alkyle, cycloalkyle, aryle, aralkyle, alkylaryle, alkyloxyalkyle, aryloxyalkyle, cycloalkoxyalkyle,
- a étant un nombre supérieur à 1 et de préférence supérieur à 2.

Les résidus aminoplastes porteurs de leurs groupements OR intégrés dans les polymères peuvent être choisis de manière non limitative parmi les structures (1) à (12) suivantes : 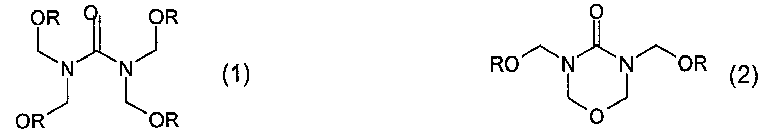 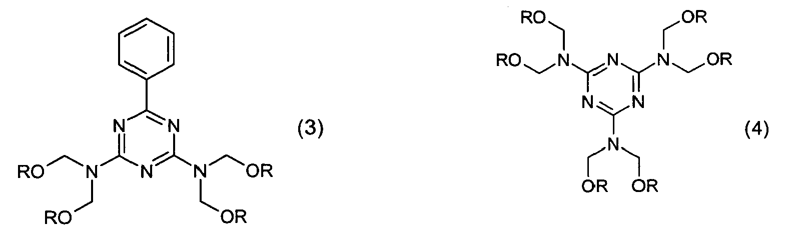 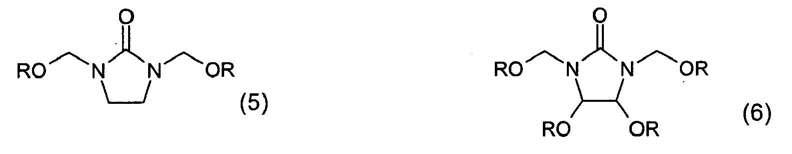 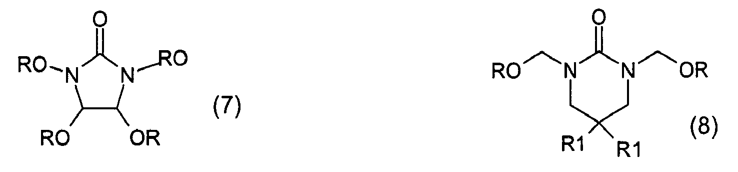 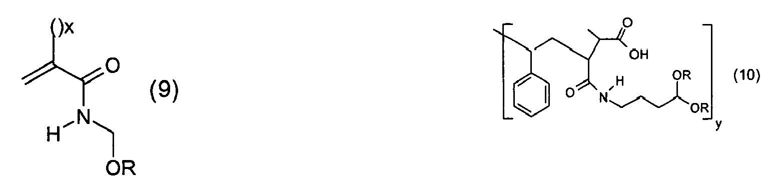 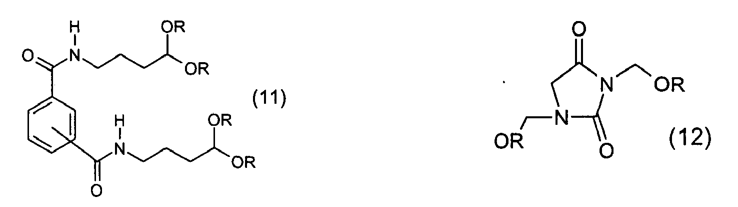 dans lesquelles :
- R a la même signification que précédemment,
- R₁ désigne alkyle C₁-C₄,
- y est un nombre au moins égal à 2,
- x désigne 0 ou 1.

De préférence, le ou les résidus aminoplastes porteurs de leurs groupements OR sont choisis parmi ceux de structure (13) suivante : 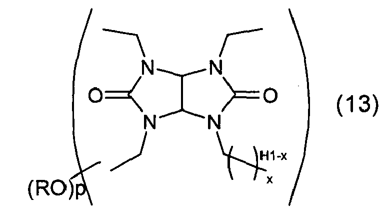 dans laquelle R, p , et x ont les mêmes significations que précédemment.

Les résidus alkylène-oxy divalents sont de préférence ceux correspondants aux diols de formule générale (14) suivante :

HO-(ZO)y-(Z1(Z2O)w)t-(Z'O)y'-Z3OH (14),

- y et y' étant des nombres allant de 0 à 1000,
- t et w étant des nombres allant de 0 à 10,
- Z, Z', Z2 et Z3 sont des radicaux alkylène en C₂-C₄ et de préférence des radicaux :
- CH2-CH(Z4)- et -CH2-CH(Z4)-CH2-,
- Z1 étant un radical linéaire ou cyclique, ramifié ou non, aromatique ou non, comportant ou non un ou plusieurs hétéroatomes et possédant de 1 à 40 atomes de carbone,
- Z4 désignant un atome d'hydrogène ou un radical alkyle en C₁-C₄ ou un radical acyle en C₁-C₃ étant entendu qu'au moins un des radicaux Z4 des radicaux Z, Z', Z2 et Z3 est différent d'un radical acyle.

De préférence Z4 désigne un atome d'hydrogène ou un radical méthyle.

Encore plus préférentiellement t=0 et Z, Z' et Z3 désignent -CH₂CH₂-, et l'un au moins de y ou y' est différent de 0. Les composés de formule (14) sont alors des polyéthylèneglycols.

Les polymères aminoplaste éther de formule (g) sont en particulier décrits dans le brevet US 5 914 373 auquel on pourra se référer pour plus de détails.

Comme polymères à squelette aminoplaste-éther de formule (g), on peut en particulier citer les produits Pure-Thix® L [PEG-180/Octoxynol-40/TMMG Copolymer (Nom INCI)], Pure-Thix M® [PEG-180/Laureth-50/TMMG Copolymer (Nom INCI)], Pure-Thix® HH [Polyether-1 (Nom INCI)] ; Pure Thix TX-1442® [copolymère PEG-18 / dodoxynol-5 / PEG-25 tristyrylphénol / tétraméthoxy méthyl glycolurile] proposés par la société Süd-Chemie.

Les polymères épaississants entrant comme ingrédient dans la composition selon l'invention peuvent aussi être choisis parmi les polymères associatifs comportant au moins un monomère à insaturation éthylénique à groupement sulfonique, sous forme libre ou partiellement ou totalement neutralisée et comprenant au moins une partie hydrophobe.

De façon préférentielle, lesdits polymères sont neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés.

Ces polymères associatifs peuvent être ou non réticulés, et de préférence sont des polymères réticulés. Dans ce cas, les agents réticulants provenant d'au moins un monomère ayant au moins deux insaturation éthyléniques (double liaison carbone-carbone).

Les monomères de réticulation ayant au moins deux insaturations éthyléniques sont choisis par exemple parmi l'éther diallylique, le triallylcyanurate, le diallylmaléate, le (méth)acrylate d'allyle, le dipropylèneglycol-diallyléther, les polyglycol-diallyléthers, le triéthylèneglycol-divinyléther, l'hydroquinone-diallyl-éther, le tétrallyl-oxéthanoyle, le di(méth)acrylate de tétra- ou di-éthylèneglycol, la triallylamine, la tétraallyléthylènediamine le triméthylolpropane-diallyléther, le triméthylolpropane triacrylate, le méthylène-bis(méth)acrylamide ou le divinylbenzène, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

On utilise plus particulièrement le méthylène-bis-acrylamide, le méthacrylate d'allyle ou le triméthylol propane triacrylate. Le taux de réticulation varie en général de 0,01 à 10% en mole, par rapport au polymère.

Les monomères à insaturation éthylénique à groupement sulfonique sont choisis notamment parmi l'acide vinylsulfonique, l'acide styrènesulfonique, les acides (méth)acrylamido(C₁-C₂₂)alkylsulfoniques, les acides N-(C₁-C₂₂)alkyl(méth)acrylamido(C₁-C₂₂)alkylsulfoniques comme l'acide undécyl-acrylamido-méthane-sulfonique ainsi que leurs formes partiellement ou totalement neutralisées.

Plus particulièrement, on peut utiliser les acides (méth)acrylamido(C₁-C₂₂) alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique ainsi que leurs formes partiellement ou totalement neutralisées.

On utilise de préférence l'acide 2-acrylamido-2-méthylpropane-sulfonique (AMPS) ainsi que ses formes partiellement ou totalement neutralisées.

Les polymères amphiphiles présents dans la composition selon l'invention peuvent aussi être choisis parmi les polymères amphiphiles statistiques d'AMPS modifiés par réaction avec une n-monoalkylamine ou une di-n-alkylamine en C₆-C₂₂, et tels que ceux décrits dans la demande WO00/31154.

Les monomères hydrophobes qui constituent la partie hydrophobe du polymère sont choisis de préférence parmi les acrylates ou les acrylamides de formule (k) suivante : 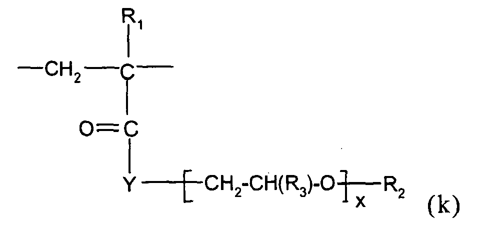 dans laquelle R₁ et R₃, identiques ou différents, désignent un atome d'hydrogène ou un radical alkyle linéaire ou ramifié en C₁-C₆ (de préférence méthyle) ; Y désigne O ou NH ; R₂ désigne un radical hydrocarboné hydrophobe tel que défini auparavant ; x désigne un nombre de moles d'oxyde d'alkylène et varie de 0 à 100.

Le radical R₂ est choisi avantageusement parmi les radicaux alkyles en C₆-C₁₈ linéaires (par exemple n-hexyle, n-octyle, n-décyle, n-hexadécyle, n-dodécyle), ramifiés ou cycliques (par exemple cyclododécane (C₁₂) ou adamantane (C₁₀)) ; les radicaux alkylperfluorés en C₆-C₁₈ (par exemple le groupement de formule -(CH₂)₂-(CF₂)₉-CF₃) ; le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle.

Selon une forme particulière de l'invention, le monomère de formule (k) comporte au moins un motif oxyde d'alkylène (x ≥1) et de préférence une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés varie en général de 3 à 100 et plus préférentiellement de 3 à 50 et encore plus préférentiellement de 7 à 25.

Les copolymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou poly-alkylèneglycols, les (méth)acrylamides, la vinylpyrrolidone, l'anhydride maléique, l'acide itaconique ou l'acide maléique ou les mélanges de ces composés.

Ces copolymères sont décrits notamment dans les documents EP750899, US 5089578, les publications de Yotaro Morishima suivantes : «Self-assembling amphiphilic polyelectrolytes and their nanostructures - Chinese Journal of Polymer Science Vol. 18, N°40, (2000), 323-336.» ; «Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704» ; «Solution properties of miscelle networks formed by non-ionic moieties covalently bound to an polyelectrolyte: salt effects on rheological behaviour- Langmuir, 2000, Vol.16, N°12, 5324-5332» ; «Stimuli responsive amphiphilic copolymers of sodium 2-(acrylamido)-2-methylpropanesulfonate and associative macromonomers - Polym. Preprint, Div. Polym. Chem. 1999, 40(2), 220-221 ».

La répartition des monomères dans le copolymère peut être statistique ou bloc.

Parmi ces polymères de ce type, on peut citer plus spécialement :
- les copolymères réticulés ou non réticulés, neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(méth)acrylate par rapport au polymère, tels que ceux décrits dans la demande EP-A750 899 ;
- les terpolymères comportant de 10 à 90% en mole de motifs acrylamide, de 0,1 à 10% en mole de motifs AMPS et de 5 à 80% en mole de motifs n-(C₆-C₁₈)alkylacrylamide, tels que ceux décrits dans le brevet US- 5089578.
- les copolymères d'AMPS totalement neutralisé et de méthacrylate de dodécyle ainsi que les copolymères d'AMPS et de n-dodécylméthacrylamide non-réticulés et réticulés, tels que ceux décrits dans les articles de Morishima cités ci-dessus.

On citera plus particulièrement les copolymères constitués de motifs AMPS de formule (1) suivante : 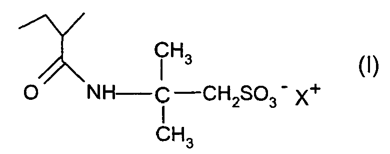 dans laquelle X+ a la même définition que précédemment,
et de motifs de formule (p) suivante : 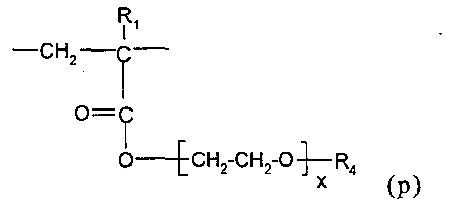 dans laquelle x désigne un nombre entier variant de 3 à 100, de préférence de 5 à 80 et plus préférentiellement de 7 à 25 ; R₁ a la même signification que celle indiquée ci-dessus dans la formule (j) et R4 désigne un alkyle linéaire ou ramifié en C₆-C₂₂ et plus préférentiellement en C₁₀-C₂₂.

Les polymères particulièrement préférés sont ceux pour lesquels x = 25, R₁ désigne méthyle et R₄ représente n-dodécyle ; ils sont décrits dans les articles de Morishima mentionnés ci-dessus.

Les polymères pour lesquels X+ désigne sodium ou ammonium sont plus particulièrement préférés.

Les polymères de la gamme Genapol® de la société Hoechst/Clariant peuvent être employés dans la composition selon l'invention.

La concentration en polymère(s) épaississant(s), associatif(s) ou non, présents dans la composition selon l'invention peut varier entre 0,01 et 10% en poids, plus particulièrement entre 0,1 à 5% en poids, par rapport au poids de la composition, et de manière encore plus avantageuse, entre 0,5 et 5 % en poids par rapport au poids de la composition.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

La composition selon l'invention peut se présenter sous des formes diverses, telles que sous forme de liquides, de shampooings, de crèmes, de gels, ou sous toute autre forme appropriée.

Une forme particulièrement préférée selon la présente invention est un shampooing colorant et/ou éclaircissant comprenant dans un milieu aqueux cosmétiquement acceptable, au moins un colorant direct tel que défini ci-dessus, et au moins un agent tensioactif .

La composition de l'invention peut renfermer au moins un agent oxydant choisi par exemple parmi le peroxyde d'hydrogène, le peroxyde d'urée, les bromates de métaux alcalins, les persels tels que les perborates et persulfates, et les enzymes telles que les peroxydases et les oxydo-réductases à deux ou à quatre électrons. L'utilisation du peroxyde d'hydrogène ou des enzymes est particulièrement préférée.

Un autre objet de l'invention est constitué par un procédé de traitement de fibres kératiniques, en particulier de fibres kératiniques humaines.

Selon une première variante, on applique sur lesdites fibres, sèches ou humides, une composition telle que définie, pendant un temps suffisant, après quoi on rince, on lave éventuellement au shampooing, on rince à nouveau et on sèche ou on laisse sécher les fibres résultantes.

Selon une deuxième variante du procédé, on applique sur lesdites fibres, sèches ou humides, une composition telle que définie sans rinçage final.

La première variante est utilisable pour tout type de compositions, que celles-ci comprennent ou non, un agent oxydant et/ou un colorant direct et/ou une base d'oxydation éventuellement associée à un coupleur.

La seconde variante est particulièrement appropriée pour des compositions ne comprenant pas de colorant d'oxydation (base d'oxydation et éventuellement coupleur) ni d'agent oxydant.

Dans le cas de la première variante du procédé, le temps d'application est habituellement suffisant pour développer la coloration et/ou l'éclaircissement souhaité(s).

A titre indicatif, la durée d'application de la composition est d'environ 5 à 60 minutes et plus particulièrement d'environ 15 à 60 minutes.

Par ailleurs, la température à laquelle le procédé selon l'invention est mis en oeuvre, est généralement comprise entre la température ambiante (15 à 25°C) et 200°C et plus particulièrement entre 15 et 60°C.

Au cas où la composition comprend un agent oxydant, le procédé selon l'invention comporte une étape préliminaire consistant à stocker sous forme séparée, d'une part, une composition comprenant, dans un milieu cosmétiquement acceptable, au moins un colorant direct correspondant à la formule (I) éventuellement au moins un colorant direct additionnel et/ou éventuellement au moins une base d'oxydation éventuellement associée à au moins un coupleur, et d'autre part, une composition comprenant, dans un milieu cosmétiquement acceptable, au moins un agent oxydant, puis à procéder à leur mélange au moment de l'emploi. Une fois ceci réalisé, le procédé selon l'invention est mis en oeuvre conformément à ce qui a été mentionné auparavant.

Un autre objet de l'invention est un dispositif à plusieurs compartiments, comprenant au moins un compartiment renfermant une composition comprenant au moins un colorant direct correspondant à la formule (I), et au moins un autre compartiment renfermant une composition comprenant au moins un agent oxydant. Ce dispositif peut être équipé d'un moyen permettant de délivrer sur les cheveux le mélange souhaité, tel que les dispositifs décrits dans le brevet FR 2586913.

A noter qu'au cas où la composition renferme au moins un colorant direct additionnel et/ou au moins une base d'oxydation éventuellement associée à au moins un coupleur, selon une première variante, ce ou ces composés se trouvent dans le premier compartiment du dispositif précédemment décrit. Selon une deuxième variante, le colorant direct additionnel et/ou la base d'oxydation/coupleur sont stockés dans un troisième compartiment.

Il est précisé qu'il ne serait pas exclu d'avoir une troisième variante combinant les deux précédentes, dans laquelle le colorant direct additionnel et/ou la base d'oxydation et éventuellement le coupleur se trouveraient en partie dans le premier compartiment, avec le colorant direct correspondant à la formule (I), et en partie dans un troisième compartiment.

Les exemples qui suivent sont destinés à illustrer l'invention sans pour autant en limiter la portée.

### Exemples de compositions selon l'invention :

| Composition colorante | |
|---|---|
| Composé de l'invention | quantité |
| Colorant direct méthinique de formule A, B, C, D ou E | 10⁻³ mol% |
| Hydroxyéthylcellulose vendue par la société Aqualon sous la dénomination Natroso1250MR | 0,384% |
| Melange de p-hydroxybenzoates de méthyle, éthyle, propyle, butyle et isobutyle vendu par la société NIPA sous la dénomination de NIPA ester 82121 | 0,032% |
| Alkyl(C8/C10 50/50)polyglucoside vendu par la société SEPPIC sous la dénomination Oramix CG110 | 5% |
| Alcool benzylique | 4% |
| Propylèneglycol (8OE) | 6% |
| Eau déminéralisée qsp | 100 |

Le composé (A) présente la structure suivante :

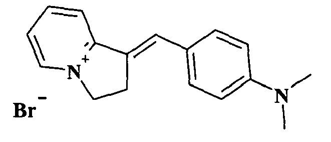

### Bromure de 1-(4-Dimethylamino-benzylidene)-2,3-dihydro-1H-indolizinylium

Cette composition colorante est appliquée sur des cheveux à 90% de blancs naturels.

La température et le temps de pause sont respectivement de 33°C et 30 minutes.

Après rinçage, shampooing et séchage au casque pendant 30 minutes, les cheveux présentent une coloration jaune orangé très esthétique.

La couleur obtenue est tenace aux shampooings. La composition est stable au stockage.

Le composé (B) a la structure suivante :

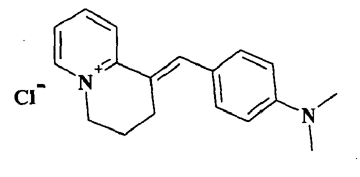

### Chlorure de 1-(4-Dimethylamino-benzylidene)-1,2,3,4-tetrahydro-quinolizinylium

Cette composition colorante est appliquée sur cheveux à 90% de blancs naturels.

La température et le temps de pause sont respectivement de 33°C et 30 minutes.

Après rinçage, shampooing et séchage au casque pendant 30 minutes, les cheveux présentent une coloration orangée très esthétique.

La couleur obtenue est tenace aux shampooings. La composition est stable au stockage.

Le composé (C) a la structure suivante :

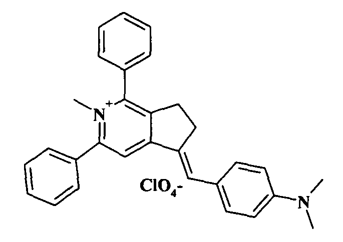

### Perchlorate de 5H-Cyclopenta[c]pyridinium, 5-[[4-(dimethylamino)phenyl]methylene]-6,7-dihydro-2-methyl-1,3-diphenyl

La composition est appliquée sur cheveux blancs naturels, pendant 20 minutes à température ambiante(25°C). Après teinture, les mèches sont rincées et séchées.

La couleur orange obtenue est tenace aux shampooings. La composition est stable au stockage.

Le composé (D) a la structure suivante :

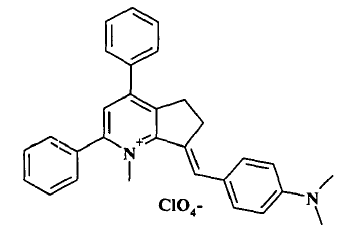

### Perchlorate de 5H-Cyclopenta[b]pyridinium, 7-[[4-(dimethylamino)phenyl]methylene]-6,7-dihydro-1-methyl-2,4-diphenyl

La composition est appliquée sur cheveux gris à 90% de blancs naturels, pendant 20 minutes à température ambiante. Après teinture, les mèches sont rincées et séchées.

La couleur jaune-orangee obtenue est tenace aux shampooings. La composition est stable au stockage.

## Revendications

1. Utilisation d'au moins un composé de formule (I) suivante 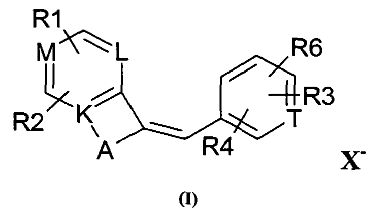 dans laquelle :
M et L représentent indépendamment l'un de l'autre CR₇, CR₉ ou N⁺R₅ ;
K désigne un atome de carbone ou d'azote quaternisé N⁺ ;
T désigne un groupement N⁺R₅, un groupement CR₄ ou un atome d'azote ;
A représente un groupe alkylène CₙH₂ₙ linéaire ou ramifié comportant de 1 à 8 atomes de carbone et éventuellement terminé à l'une de ses extrémités par un atome d'oxygène ou un groupement carbonyle ; un groupe comprenant au moins une insaturation, l'insaturation faisant partie d'un noyau benzénique ou d'un hétérocycle éventuellement substitué par un ou plusieurs radicaux alkyle C₁C₄ ou aryle C₆C_{30 ;}
R₁, R₂, R₃, R₄, R₆, R₇ et R₉, indépendamment les uns des autres, représentent un atome d'hydrogène ; un atome d'halogène ; un groupement aryle en C₆-C₃₀ ; un groupement hydroxy ; un groupement cyano ; un groupement nitro ; un groupement sulfo ; un groupement amino ; un groupement acylamino ; un groupement dialkyl(C₁-C₆)amino ; un groupement dihydroxyalkyl(C₁-C₆)amino ; un groupement alkyl(C₁-C₆)hydroxyalkyl(C₁-C₆)amino ; un groupement alcoxy(C₁-C₆) ; un groupement alcoxy(C₁-C₆)carbonyle ; un groupement carboxyalcoxy en C₁-C₆ ; un groupement pipéridinosulfonyle ; un groupement pyrrolidino ; un groupement alkyle(C₁-C₆)halogénoalkyle(C₁-C₆)amino ; un groupement benzoylalkyle(C₁-C₆) ; un groupement vinyle ; un groupement formyle ; un radical aryle en C₆-C₃₀ éventuellement substitué par un ou plusieurs groupements choisis parmi les groupements hydroxyle, alcoxy en C₁-C₆ linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C₁-C₆; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C₁-C₆ linéaire, ramifié ou cyclique, , aryle éventuellement substitué, carboxyle, sulfo, un atome d'halogène,ce radical alkyle pouvant être interrompu par un hétéroatome, tel que N, S ou O ;
Deux des substituants R₁, R₂, R₇ et R₉, lorsque M et L représentent respectivement CR₇, CR₉, peuvent former avec les atomes de carbone auxquels ils sont rattachés un cycle aromatique ou non, en C₆-C₃₀ ou hétérocyclique comportant au total de 5 à 30 chaînons et de 1 à 5 hétéroatomes ; ces cycles étant condensés ou non, insérant ou non un groupement carbonyle, et étant substitués ou non par un ou plusieurs groupements choisis parmi les groupements alkyle en C₁-C₄, alcoxy(C₁-C₄)alkyl(C₁-C₄), amino, dialkyl(C₁-C₄)amino, halogène, phényle, carboxy, trialkyl(C₁-C₄)ammonioalkyl(C₁-C₄) ;
R₁, R₂ et R₇ ou R₉ peuvent également représenter un groupe similaire à la partie de la molécule condensée sur le noyau pyridinium de manière à former une molécule parfaitement symétrique, comportant ainsi nécessairement un enchaînement acridinium ;
Deux des substituants R₃, R₄ et R₆ peuvent former avec les atomes de carbone auxquels ils sont rattachés un noyau aromatique en C₆-C₃₀ ou hétérocyclique comportant au total de 5 à 30 chaînons et de 1 à 5 hétéroatomes ; ce cycle étant condensés ou non, ce cycle et l'éventuel cycle condensé étant substitués ou non par au moins un groupement choisi parmi les groupements alkyle en C₁-C₄, alcoxy(C₁-C₄)alkyl(C₁-C₄), amino, dialkyl(C₁-C₄)amino, halogène, phényle, carboxy, trialkyl(C₁-C₄)ammonioalkyl(C₁-C₄), ce cycle peut aussi former avec le troisième substituant et l'atome de carbone le portant un nouveau cycle comportant de 5 à 10 chaînons, saturé ou insaturé ;
R₅ représente un radical alkyle linéaire, ramifié ou cyclique, comprenant de 1 à 22 atomes de carbone, éventuellement substitué par au moins un groupement choisi parmi les groupements hydroxyle, alcoxy en C₁-C₆ linéaire, ramifié ou cyclique, aryle éventuellement substitué, carboxyle, sulfo, un atome d'halogène, ce radical alkyle étant éventuellement interrompu par un hétéroatome tel que N, O ou S ;un radical aryle C₆C₃₀ éventuellement substitué par un ou plusieurs radicaux alkyle C₁C₄, alcoxy C₁C₄, hydroxyle ;
R₅ peut former avec l'atome d'azote le portant et avec un des substituants R₁, R₂, R₇, R₉ et l'atome de carbone portant ce substituant un cycle de 5 à 10 chaînons substitué ou non par un groupement alkyle. Ce cycle peut être condensé éventuellement avec un cycle benzénique ou avec un cycle formé par deux des substituants restants et les atomes de carbone les portant ;
X⁻ représente un anion organique ou minéral ;
un seul de K, L, M désigne nécessairement N⁺R₅ ou N⁺ ;
en tant qu'agent colorant des fibres kératiniques, en particulier humaines, et/ou en tant qu'agent pour l'éclaircissement optique de ces fibres.

2. Utilisation selon la revendication 1, **caractérisée en ce que** R₁, R₂, R₃, R₄, R₆, R₇ et R₉ indépendamment les uns des autres, représentent un atome d'hydrogène ; un groupement aryle en C₆-C₃₀ éventuellement substitué par un groupement choisis parmi les radicaux alkyle en C₁-C₆, alcoxy en C₁-C₆ et hydroxyle ; un groupement hydroxy ; un groupement nitro ; un groupement vinyle ; un groupement pyrrolidino ; un groupement benzoylalkyle(C₁-C₆) ; un groupement alkyl(C₁-C₆)halogénoalkyle(C₁-C₆)amino ; un groupement amino ; un groupement dialkyl(C₁-C₆)amino ; un groupement dihydroxyalkyl(C₁-C₆)amino ; un groupement alkyl(C₁-C₆)hydroxyalkyl(C₁-C₆)amino ; un groupement alcoxy(C₁-C₆) ; un groupement formyle ; un radical alkyle linéaire, ramifié ou cyclique, comprenant de 1 à 6 atomes de carbone, éventuellement substitué par un ou plusieurs groupements choisis parmi les groupements hydroxyle aryle éventuellement substitué,
R₁, R₂, R₇ et R₉ peuvent former avec les atomes de carbone auxquels ils sont rattachés un cycle aromatique ou non, en C₆-C₃₀ ou hétérocyclique comportant au total de 5 à 30 chaînons et de 1 à 5 hétéroatomes ; ces cycles étant condensés ou non avec insertion ou non d'un groupement carbonyle, et étant substitués ou non par un ou plusieurs groupements choissi parmi les groupements alkyle en C₁-C₄, alcoxy(C₁-C₄)alkyl(C₁-C₄), amino, dialkyl(C₁-C₄)amino, phényle,
R₁, R₂, R₇ et R₉ peuvent également représenter un groupe similaire à la partie de la molécule condensée sur le noyau pyridinium de manière à former une molécule parfaitement symétrique, comportant ainsi nécessairement un enchaînement acridinium ;
R₃ et R₄ peuvent former avec les atomes de carbone auxquels ils sont rattachés un noyau aromatique en C₆-C₃₀ ou hétérocyclique comportant au total de 5 à 30 chaînons et de 1 à 5 hétéroatomes ; ces cycles étant condensés ou non substitués ou non par au moins un groupement alkyle en C₁-C₄ ; ce cycle peut aussi former avec R6 et l'atome de carbone le portant un nouveau cycle comportant de 5 à 10 chaînons, saturé ou insaturé ;
R₅ représente un radical alkyle linéaire, ramifié ou cyclique, comprenant de 1 à 22 atomes de carbone, un radical aryle C₆C₃₀ éventuellement substitué par un ou plusieurs radicaux alkyle C₁C₄, alcoxy C₁C₄, hydroxyle ;
R₅ peut former avec l'atome d'azote le portant et avec R₁ et l'atome de carbone portant ce substituant R₁, un cycle de 5 à 10 chaînons substitué ou non par un groupement alkyle ; ce cycle peut être condensé éventuellement avec un cycle benzénique ou avec un cycle formé par R₁ et R₂ et les atomes de carbone les portant.

3. Utilisation selon la revendication 1, **caractérisée en ce que** les colorants de formule (I) sont choisis parmi les composés de formules (II) à (V) suivantes : 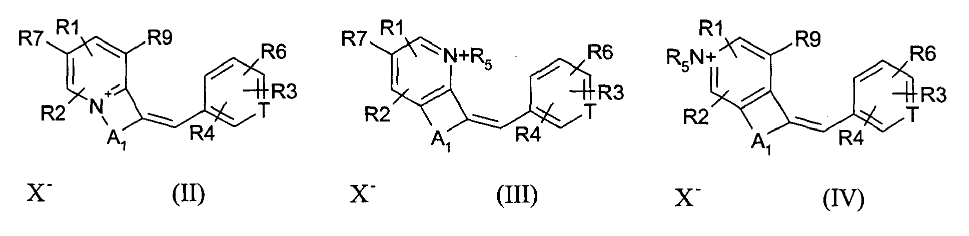 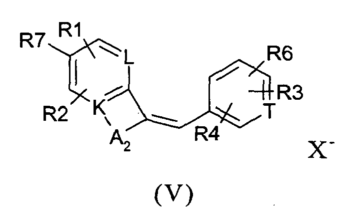 dans lesquelles :
R₁, R₂, R₃, R₄, R₅, R₆, R₇, R₉, K, L, X et T ont les mêmes significations que dans le cas de la formule (I) et dans lesquelles:
A₁ représente un groupe alkylène CₙH₂ₙ linéaire ou ramifié comportant de 1 à 8 atomes de carbone et éventuellement terminé à l'une de ses extrémités par un atome d'oxygène ou un groupement carbonyle ;
A₂ représente un groupe comprenant au moins une insaturation, l'insaturation faisant partie d'un noyau benzénique ou d'un hétérocycle éventuellement substitué par un ou plusieurs radicaux alkyle C₁C₄ ou aryle C₆C₃₀ ;
un seul des groupements K ou L désignant N⁺ ou un groupe N⁺R₅.

4. Utilisation selon la revendication 3, **caractérisée en ce que** dans la formule (II),
A₁ représente un groupe alkylène CₙH₂ₙ linéaire ou ramifié comportant de 1 à 8 atomes de carbone et éventuellement terminé à l'une de ses extrémités par un groupement carbonyle ;
R₁, R₂, R₃, R₄, R₆, R₇ et R₉, indépendamment les uns des autres représentent un atome d'hydrogène ; un groupement aryle en C₆-C₃₀ éventuellement substitué par un groupement choisis parmi les radicaux alkyle en C₁-C₆, alcoxy en C₁-C₆ et hydroxyle ; un groupement hydroxy ; un groupement vinyle ; un groupement pyrrolidino ; un groupement benzoylalkyle(C₁-C₆) ; un groupement alkyl(C₁-C₆)halogénoalkyle(C₁-C₆)amino ; un groupement amino ; un groupement dialkyl(C₁-C₆)amino ; un groupement dihydroxyalkyl(C₁-C₆)amino ; un groupement alkyl(C₁-C₆)hydroxyalkyl(C₁-C₆)amino ; un groupement alcoxy(C₁-C₆) ; un groupement formyle ; un radical alkyle linéaire, ramifié ou cyclique, comprenant de 1 à 6 atomes de carbone, éventuellement substitué par un ou plusieurs groupement choisis parmi les groupements hydroxyle aryle éventuellement substitué,
R₃ et R₄ peuvent former avec les atomes de carbone auxquels ils sont rattachés un noyau aromatique en C₆-C₃₀ ou hétérocyclique comportant au total de 5 à 30 chaînons et de 1 à 5 hétéroatomes ; ces cycles étant condensés ou non substitués ou non par au moins un groupement alkyle en C₁-C₄ ; ce cycle peut aussi former avec R₆ et l'atome de carbone le portant un nouveau cycle comportant de 5 à 10 chaînons, saturé ou insaturé.

5. Utilisation selon la revendication 4, **caractérisée en ce que**
A₁ représente un groupement alkylène comprenant de 2 à 4 atomes de carbone ;
T désigne CR₄, N⁺R₅ ou N ;
R₁, R₂, R₇ et R₉ désignent un atome d'hydrogène ;
R₃ et R₄ désignent indépendamment l'un de l'autre un atome d'hydrogène ; un radical alkyle en C₁-C₄ (de préférence méthyle) ; un radical alcoxy en C₁-C₄ (de préférence méthoxy), un groupement dialkyl(C₁-C₄)amino (de préférence diméthylamino ou diéthylamino) ; un groupement dihydroxyalkyl(C₁-C₄)amino (de préférence dihydroxyéthylamino); un groupement alkyl(C₁-C₄)hydroxyalkyl(C₁-C₄)amino (de préférence méthylhydroxyéthylamino) ; un groupement pyrrolidino, un groupe formyle ou forment ensemble avec les atomes de carbone les portant un cycle à 5 ou 6 chaînons comportant éventuellement un ou plusieurs hétéroatomes, ce cycle étant éventuellement condensé, et l'ensemble étant éventuellement substitué par un ou plusieurs radicaux alkyle en C₁-C₆ ;
R₆ désigne un atome d'hydrogène ou forme un cycle incluant un chaînon d'un cycle formé par R₃ et/ou R₄ ;

6. Utilisation selon la revendication 3, **caractérisée en ce que** dans la formule (III),
A₁ représente un groupe alkylène CₙH₂ₙ linéaire ou ramifié comportant de 1 à 8 atomes de carbone et éventuellement terminé à l'une de ses extrémités par un groupement carbonyle ;
R₁, R₂, R₃, R₄, R₆, et R₇ indépendamment les uns des autres représentent un atome d'hydrogène ; un groupement aryle en C₆-C₃₀ éventuellement substitué par un groupement choisis parmi les radicaux alkyle en C₁-C₆, alcoxy en C₁-C₆ et hydroxyle ; un groupement hydroxy ; un groupement vinyle ; un groupement pyrrolidino ; un groupement benzoylalkyle(C₁-C₆) ; un groupement alkyl(C₁-C₆)halogénoalkyle(C₁-C₆)amino ; un groupement amino ; un groupement dialkyl(C₁-C₆)amino ; un groupement dihydroxyalkyl(C₁-C₆)amino ; un groupement alkyl(C₁-C₆)hydroxyalkyl(C₁-C₆)amino ; un groupement alcoxy(C₁-C₆) ; un radical alkyle linéaire, ramifié ou cyclique, comprenant de 1 à 6 atomes de carbone, éventuellement substitué par un ou plusieurs groupement choisis parmi les groupements hydroxyle aryle éventuellement substitué ;
R₃ et R₄ peuvent former avec les atomes de carbone auxquels ils sont rattachés un noyau aromatique en C₆-C₃₀ ou hétérocyclique comportant au total de 5 à 30 chaînons et de 1 à 5 hétéroatomes ; ces cycles étant condensés ou non substitués ou non par au moins un groupement alkyle en C₁-C₄. Ce cycle peut aussi former avec R6 et l'atome de carbone le portant un nouveau cycle comportant de 5 à 10 chaînons, saturé ou insaturé ;
R₁ et R₇ peuvent également représenter un groupe similaire à la partie de la molécule condensée sur le noyau pyridinium de manière à former une molécule parfaitement symétrique, comportant ainsi nécessairement un enchaînement acridinium ;
R₅ représente un radical alkyle linéaire, ramifié ou cyclique, comprenant de 1 à 10 atomes de carbone, un radical aryle C₆C₃₀ éventuellement substitué par un ou plusieurs radicaux alkyle C₁C₄, alcoxy C₁C₄, hydroxyle ;
R₅ peut former avec l'atome d'azote le portant et avec R₁ et l'atome de carbone portant ce substituant R₁ un cycle de 5 à 10 chaînons substitué ou non par un groupement alkyle ; ce cycle peut être condensé éventuellement avec un cycle benzénique ou avec un cycle formé par R₁ et R₂ et les atomes de carbone les portant.

7. Utilisation selon la revendication 6, **caractérisée en ce que** :
A₁ représente un groupement alkylène comprenant de 2 à 5 atomes de carbone, et éventuellement terminé à l'une de ses extrémités par un groupement carbonyle , et éventuellement substitué par au moins un groupe alkyle en C₁C₆ ;
T désigne CR₄ ;
R₁, R₂ et R₇ indépendamment les uns des autres représentent un atome d'hydrogène, un radical phényle ou forment ensemble avec les atomes de carbone les portant un cycle aromatique en C₆-C₁₂ ou un cycle saturé insérant éventuellement un groupe carbonyle dans ses chaînons, ces cycles étant éventuellement substitués par des groupements alkyle en C₁C₄ (de préférence méthyle) ;
R₅ désigne un radical alkyle en C₁C₄ (de préférence méthyle ou éthyle) ; un radical aryle C₆C₁₂ éventuellement substitué par un radical alkyle en C₁C₄, hydroxy, alcoxy en C₁C₄ (de préférence méthoxy) ; ou forme un cycle incluant un chaînon d'un cycle issu de R₁, R₂ et/ou R₇ et des atomes les portant ;
R₃ et R₆ désignent un atome d'hydrogène ;
R₄ désigne un atome d'hydrogène, un groupement dialkyl(C₁-C₄)amino (de préférence diméthylamino) ou un groupement alkyl(C₁-C₄)halogénoalkyle(C₁-C₄)amino (de préférence éthylchloroéthylamino).

8. Utilisation selon la revendication 3, **caractérisée en ce que** dans la formule (IV) :
A₁ représente un groupe alkylène CₙH₂ₙ linéaire ou ramifié comportant de 1 à 8 atomes de carbone et éventuellement terminé à l'une de ses extrémités par un groupement carbonyle ;
R₁, R₂, R₃, R₄, R₆ et R₉ indépendamment les uns des autres représentent un atome d'hydrogène ; un groupement aryle en C₆-C₃₀ éventuellement substitué par un groupement choisis parmi les radicaux alkyle en C₁C₆, alcoxy en C₁C₆ et hydroxyle; un groupement hydroxy ; un groupement vinyle ; un groupement pyrrolidino ; un groupement benzoylalkyle(C₁-C₆) ; un groupement alkyl(C₁-C₆)halogénoalkyle(C1-C6)amino ; un groupement amino ; un groupement dialkyl(C₁-C₆)amino ; un groupement dihydroxyalkyl(C₁-C₆)amino ; un groupement alkyl(C₁-C₆)hydroxyalkyl(C₁-C₆)amino ; un groupement alcoxy(C₁-C₆) ; un radical alkyle linéaire, ramifié ou cyclique, comprenant de 1 à 6 atomes de carbone, éventuellement substitué par un ou plusieurs groupements choisis parmi les groupements hydroxyle aryle éventuellement substitué ;
R₃ et R₄ peuvent former avec les atomes de carbone auxquels ils sont rattachés un noyau aromatique en C₆-C₃₀ ou hétérocyclique comportant au total de 5 à 30 chaînons et de 1 à 5 hétéroatomes ; ces cycles étant condensés ou non substitués ou non par au moins un groupement alkyle en C₁-C₄ ; ce cycle peut aussi former avec R6 et l'atome de carbone le portant un nouveau cycle comportant de 5 à 10 chaînons, saturé ou insaturé ;
R₅ représente un radical alkyle linéaire, ramifié ou cyclique, comprenant de 1 à 22 atomes de carbone, un radical aryle C₆C₃₀ éventuellement substitué par un ou plusieurs radicaux alkyle C₁C₄, alcoxy C₁C₄, hydroxyle ;
R₅ peut former avec l'atome d'azote le portant et avec R₁ et l'atome de carbone portant ce substituant R₁ un cycle de 5 à 10 chaînons substitué ou non par un groupement alkyle ; ce cycle peut être condensé éventuellement avec un cycle benzénique ou avec un cycle formé par R₁ et R₂ et les atomes de carbone les portant.

9. Utilisation selon la revendication 8, **caractérisée en ce que** :
A₁ représente un groupe alkylène CₙH₂ₙ linéaire ou ramifié comportant de 1 à 4 atomes de carbone ;
T désigne CR₄ ;
R₂, R₃ et R₆ représentent un atome d'hydrogène ;
R₁ et R₉ représentent indépendamment l'un de l'autre un atome d'hydrogène, un radical aryle en C₆C₁₂ ou forment ensemble avec les atomes de carbone qui les portent, un cycle aromatique éventuellement condensé(s) en C₆-C₁₂, tous ces cycles pouvant éventuellement être substitués;
R₄ représente un atome d'hydrogène, un groupement dialkyl (C₁-C₆)amino (de préférence diméthylamino, diéthylamino ou dihexylamino), un groupement dihydroxyalkyl(C₁-C₆)amino, un groupement alkyl(C₁-C₆)hydroxyalkyl(C₁-C₆)amino ;
R₅ représente un groupe alkyle en C₁-C₂₂, phényle, benzyle, ou forme avec R₁ et R₂ et/ou R₉ et les atomes les portant deux cycles accolés.

10. Utilisation selon la revendication 3, **caractérisée en ce que** dans la formule (V) :
A₂ représente un groupe comprenant au moins une insaturation, l'insaturation faisant partie d'un noyau benzénique ou d'un hétérocycle éventuellement substitué par un ou plusieurs radicaux alkyle C₁C₄ ou aryle C₆C₃₀ ;
R₁, R₂, R₃, R₄, R₆ et R₇ indépendamment les uns des autres représentent un atome d'hydrogène ; un groupement aryle en C₆-C₃₀ éventuellement substitué par un groupement choisis parmi les radicaux alkyle en C₁C₆, alcoxy en C₁C₆ et hydroxyle ; un groupement hydroxy ; un groupement vinyle ; un groupement pyrrolidino ; un groupement benzoylalkyle(C₁-C₆) ; un groupement alkyl(C₁-C₆)halogénoalkyle(C₁-C₆)amino ; un groupement amino ; un groupement dialkyl(C₁-C₆)amino ; un groupement dihydroxyalkyl(C₁-C₆)amino ; un groupement alkyl(C₁-C₆₎hydroxyalkyl(C₁-C₆)amino ; un groupement alcoxy(C₁-C₆) ; un groupement formyle ; un radical alkyle linéaire, ramifié ou cyclique, comprenant de 1 à 6 atomes de carbone, éventuellement substitué par un ou plusieurs groupements choisis parmi les groupements hydroxyle aryle éventuellement substitué ;
R₃ et R₄ peuvent former avec les atomes de carbone auxquels ils sont rattachés un noyau aromatique en C₆-C₃₀ ou hétérocyclique comportant au total de 5 à 30 chaînons et de 1 à 5 hétéroatomes ; ces cycles étant condensés ou non substitués ou non par au moins un groupement alkyle en C₁-C₄ ; ce cycle peut aussi former avec R6 et l'atome de carbone le portant un nouveau cycle comportant de 5 à 10 chaînons, saturé ou insaturé ;

11. Utilisation selon la revendication 10, **caractérisée en ce que**
R₁, R₂ et R₇ désignent indépendamment les uns des autres un atome d'hydrogène, un radical alkyle en C₁C₄, vinyle, ou peuvent former ensemble avec les atomes de carbone les portant un cycle saturé ou insaturé portant éventuellement un ou plusieurs radicaux alkyle ;
R₆ représente un atome d'hydrogène ;
R₃ et R₄ représentent indépendamment l'un de l'autre un atome d'hydrogène ; un groupe alkyle C₁-C₄ (de préférence méthyle) ; un groupe dialkylamino (de préférence diméthylamino) ; un groupe alcoxy C₁-C₄ (de préférence méthoxy, éthoxy) ; un radical hydroxy ; un groupe nitro ; un radical benzoylalkyle C₁-C₄ ; ou forment ensemble un cycle à 5 ou 6 chaînons avec les atomes de carbone qui les portent.
Si L représente N⁺R₅, R₅ désigne un radical alkyle C₁-C₄ ou un radical benzoylalkyleC₁-C₄.
Si K représente N⁺, R₉ désigne un atome d'hydrogène.

12. Utilisation selon la revendication 11, **caractérisée en ce que** A₂ désigne un enchaînement -C(R₁₀)=C(R₁₁)-, un enchaînement -N=CH-NH- ou un radical pyrazolyle ;
R₁₀, R₁₁ désignant indépendamment l'un de l'autre un radical alkyle en C₁-C₄ (de préférence méthyle), un radical phényle ou forment ensemble un noyau benzénique.

13. Utilisation selon l'une quelconque des revendications 1 à 12, **caractérisée en ce que** l'anion d'origine minérale est choisi parmi les halogénures, les sulfates, les bisulfates, les nitrates, les phosphates, les hydrogénophosphates, les dihydrogénophosphates, les boronates, les carbonates, les bicarbonates ; et **en ce que** l'anion d'origine organique, est choisi parmi ceux provenant de sels d'acides mono- ou polycarboxyliques, sulfoniques, sulfuriques, saturés ou non, aromatiques ou non, éventuellement substitués par au moins un radical hydroxyle, amino, ou atomes d'halogène.

14. Utilisation selon la revendication précédente, **caractérisée en ce que** l'anion est choisi parmi le chlorure, l'iodure, le sulfate, le méthosulfate, l'éthosulfate.

15. Utilisation selon l'une quelconque des revendications 1 à 14, **caractérisée en ce que** les composés sont choisis parmi :
- perchlorate de Benzo[c]quinolizinium, 4-[[4-(dimethylamino)phenyl]methylene]-1,2,3,4-tetrahydro- ;
- chlorure de 1-(4-Dimethylamino-benzylidene)-1,2,3,4-tetrahydro-quinolizinylium ;
- chlorure de 1-(2,3,6,7-Tetrahydro-1H,5H-pyrido[3,2,1-ij]quinolin-9-ylmethylene)-1,2,3,4-tetrahydro-quinolizinylium
- bromure de 1-(4-Dimethylamino-benzylidene)-2,3-dihydro-1H-indolizinylium ;
- bromure de 1-(2,3,6,7-Tetrahydro-1H,5H-pyrido[3,2,1-ij]quinolin-9-ylmethylene)-2, 3-dihydro-1H-indolizinylium
- bromure de 1-(1-Methyl-1,2,3,4-tetrahydro-quinolin-6-ylmethylene)-2,3-dihydro-1H-indolizinylium ;
- bromure de 1-(1-Ethyl-2,3-dihydro-1H-indol-5-ylmethylene)-2,3-dihydro-1H-indolizinylium ;
- dibromure de 1-(4-Pyrrolidin-1-yl-benzylidene)-2,3-dihydro-1 H-indolizinylium ;
- bromure de 1-(4-Dimethylamino-naphthalen-1-ylmethylene)-2,3-dihydro-1H-indolizinylium;
- bromure de 1-(9-Ethyl-9H-carbazol-3-ylmethylene)-2,3-dihydro-1H-indolizinylium ;
- bromure de 1-(4-Methyl-3,4-dihydro-2H-benzo[1, 4]oxazin-7-ylmethylene)-2,3-dihydro-1H-indolizinylium ;
- bromure de 1-Pyridin-4-ylmethylene-2,3-dihydro-1H-indolizinylium
- bromure de 1-(4-Dimethylamino-2-methoxy-benzylidene)-2,3-dihydro-1H-indolizinylium ;
- bromure de 1-Naphthalen-2-ylmethylene-2,3-dihydro-1H-indolizinylium ;
- bromure de 1-(1-Isopropyl-1,2,3,4-tetrahydro-quinolin-6-ylmethylene)-2,3-dihydro-1H-indolizinylium ;
- bromure de 1-Naphthalen-1-ylmethylene-2,3-dihydro-1H-indolizinylium ;
- bromure de 1-{4-[Ethyl-(2-hydroxy-ethyl)-amino]-2-methylbenzylidene}-2,3-dihydro-1H-indolizinylium ;
- bromure de 1-(4-Formyl-benzylidene)-2,3-dihydro-1H-indolizinylium;
- bromure de 1-{4-[Bis-(2-hydroxy-ethyl)-amino]-benzylidene}-2,3-dihydro-1H-indolizinylium ;
- bromure de 1-(1,1,7,7-Tetramethyl-2,3,6,7-tetrahydro-1H,5H-pyrido[3,2,1-ij]quinolin-9-ylmethylene)-2,3-dihydro-1H-indolizinylium
- bromure de 1-(9H-Fluoren-2-ylmethylene)-2,3-dihydro-1H-indolizinylium ;
- bromure de 1-(4-Dimethylamino-2-methyl-benzylidene)-2,3-dihydro-1H-indolizinylium ;
- bromure de 1-(2,2-Dimethyl-chroman-6-ylmethylene)-2,3-dihydro-1H-indolizinylium ;
- bromure de 1-Methyl-Pyridinium-4-ylmethylene-2,3-dihydro-1H-indolizinylium
- bromure de 1-(1-Isobutyl-2,2-dimethyl-2,3-dihydro-1H-indol-5-ylmethylene)-2,3-dihydro-1H-indolizinylium ;
- bromure de 1-Pyren-1-ylmethylene-2,3-dihydro-1H-indolizinylium;
- bromure de 1-(4-Diethylamino-benzylidene)-2,3-dihydro-1H-indolizinylium ;
- bromure de 1-Quinolin-4-ylmethylene-2,3-dihydro-1H-indolizinylium
- chlorure de Acridinium, 4,5-bis[[4-(dimethylamino)phenyl]methylene]-1,2,3,4,5,6,7,8-octahydro-3,3,6,6-tetramethyl-1,8-dioxo-10-phenyl ;
- Sel de 5H-Cyclopenta[b]pyridinium, 7-[[4-(dimethylamino)phenyl]methylene]-6,7-dihydro-1-methyl-2,4-diphenyl;
- sulfate de 3-Benzylidene-2,3-dihydro-4-methyl-1H-cyclopenta[b]quinolinium methyl ;
- iodure de Cycloocta[b]pyridinium, 5,6,7,8,9,10-hexahydro-1-methyl-10-(phenylmethylene)- ;
- perchlorate de 1H-Pyrido[3,2,1-de]acridinium, 11-[[4-(dimethylamino)phenyl]methylene]-2,3,8,9,10,11-hexahydro-10,10-dimethyl-8-oxo- ;
- perchlorate d'Acridinium, 4-[[4-(dimethylamino)phenyl]methylene]-1,2,3,4-tetrahydro-3,3,10-trimethyl-1-oxo ;
- perchlorate de 1H-Indolo[1,2-a]quinolinium, 2,3,4,7-tetrahydro-7,7-dimethyl-5-phenyl-1-(phenylmethylene)- ;
- chlorure d'Acridinium, 4-[[4-(dimethylamino)phenyl]methylene]-1,2,3,4,5,6,7,8-octahydro-3,3,6,6-tetramethyl-1,8-dioxo-10-phenyl ;
- iodure d'Acridinium, 4-[[4-[(2-chloroethyl)ethylamino]phenyl]methylene]-1,2,3,4-tetrahydro-9,10-dimethyl
- bromure de Benz[b]acridinium, 4-[[4-(dimethylamino)phenyl]methylene]-1,2,3,4-tetrahydro-3,3-dimethyl-5-(4-methylphenyl)-1-oxo ;
- bromure de Benz[b]acridinium, 4-[[4-(dimethylamino)phenyl]methylene]-1,2,3,4-tetrahydro-1-oxo-5-phenyl ;
- perchlorate d'Acridinium, 4-[[4-(dimethylamino)phenyl]methylene]-1,2,3,4-tetrahydro-10- (4-hydroxyphenyl)-3,3-dimethyl-1-oxo ;
- perchlorate d'Acridinium, 4-[[4-(dimethylamino)phenyl]methylene]-1,2,3,4-tetrahydro-3,3-dimethyl-1-oxo-10-phenyl ;
- perchlorate d'Acridinium, 4-[[4-(dimethylamino)phenyl]methylene]-10-ethyl-1,2,3,4-tetrahydro-3,3-dimethyl-1-oxo ;
- iodure de 1H-Cyclopenta[b]quinolinium, 3-[p-(dimethylamino)benzylidene]-2,3-dihydro-4,9-dimethyl
- chlorure d'Acridinium, 4-[[4-(dimethylamino)phenyl]methylene]-1,2,3,4,5,6,7,8-octahydro-3,3,6,6-tetramethyl-10-(4-methylphenyl)-1,8-dioxo ;
- chlorure d'Acridinium, 4-[[4-(dimethylamino)phenyl]methylene]-1,2,3,4,5,6,7,8-octahydro-3,3,6,6-tetramethyl-10-(1-naphthalenyl)-1,8-dioxo- ;
- bromure de Benz[b]acridinium, 4-[[4-(dimethylamino)phenyl]methylene]-1,2,3,4-tetrahydro-S-(4-methoxyphenyl)-3,3-dimethyl-1-oxo ;
- perchlorate d'Acridinium, 4-[[4-(dimethylamino)phenyl]methylene]-1,2,3,4-tetrahydro-1-oxo-10-phenyl ;
- bromure de Benz[b]acridinium, 4-[[4-(dimethylamino)phenyl]methylene]-1,2,3,4-tetrahydro-3,3-dimethyl-1-oxo-5-phenyl- ;
- iodure d' Acridinium, 4-[p-(dimethylamino)benzylidene]-1,2,3,4-tetrahydro-9,10-dimethyl
- sel de 5-[[4-(dibutylamino)phenyl]methylene]-5,6,7,8-tetrahydro-2-octadecyl-(5E)-isoquinolinium ;
- sel de Isoquinolinium, 5-[[4-(diethylamino) phenyl]methylene]-5,6,7,8-tetrahydro-2-octadecyl-, (5E)- ;
- sulfate d' Isoquinolinium, 5-[[4-(dimethylamino)phenyl]methylene]-5,6,7,8-tetrahydro-2-octadecyl-, (5E) ;
- perchlorate de 4H-Pyrido[3,2,1-de]phenanthridinium, 12-[[4-(dimethylamino)phenyl]methylene]-5,6,9,10,11,12-hexahydro ;
- perchlorate de Benzo[c]phenanthridinium, 10-[[4-(dimethylamino)phenyl]methylene]-7,8,9,10-tetrahydro-5-(phenylmethyl)- ;
- perchlorate de Benzo[a]phenanthridinium, 1-[[4-(dimethylamino)phenyl]methylene]-1,2,3,4-tetrahydro-6-phenyl- ;
- perchlorate de Phenanthridinium, 10-[[4-(dimethylamino)phenyl]methylene]-7,8,9,10-tetrahydro-5-(phenylmethyl) ;
- perchlorate de Phenanthridinium, 10-[[4-(dimethylamino)phenyl]methylene]-7,8,9,10-tetrahydro-5-methyl ;
- Sel de 5H-Cyclopenta[c]pyridinium, 5-[[4-(dimethylamino)phenyl]methylene]-6,7-dihydro-2-methyl-1,3-diphenyl;
- Sel de 5-[[4-(dihexylamino)phenyl]methylene]-5,6,7,8-tetrahydro-2-octadecyl-(5E)-isoquinolinium ;
- perchlorate de 10-[[4-(dimethylamino)phenyl]methylene]-7,8,9,10-tetrahydro-5-phenyl-phenanthridinium ;
- perchlorate de 10-[[4-(dimethylamino)phenyl]methylene]-7,8,9,10-tetrahydro-5-phenyl-benzo[c]phenanthridinium ;
- perchlorate de 10-[[4-(dimethylamino)phenyl]-methylene]-5-ethyl-7,8,9,10-tetrahydro-phenanthridinium ;
- trifluoroacetate de 12-[(4-nitrophenyl)methylene]-12H-indolo[2,1-a]isoquinolinium ;
- trifluoroacetate de 12-[(4-methoxyphenyl)methylene]-12H-indolo[2,1-a]isoquinolinium ;
- trifluoroacetate de 12-[(1,3-benzodioxol-5-ylmethylene]-12H-indolo[2,1-a]isoquinolinium ;
- trifluoroacetate de 12-[(3,4-dimethoxyphenyl)methylene]-12H-indolo[2,1-a]isoquinolinium ;
- trifluoroacetate de 12-[(2-hydroxyphenyl)methylene]-12H-indolo[2,1-a]isoquinolinium ;
- nitrate de 1-(p-Dimethylaminobenzylidene)-2,3-dimethyl-1H-indolizinium ;
- perchlorate de 1-[[4-(dimethylamino)phenyl]methylene]-6-ethyl-2,3-dimethyl-1H-indolizinium ;
- chlorure de 1-(3-ethoxy-4-hydroxybenzylidene)-6-ethyl-2,3-dimethyl-1H-indolizinium ;
- sel interne d'hydroxyde de 3-(3-ethoxy-4-hydroxybenzylidene)-6-ethyl-1,2-dimethyl-3H-indolizinium;
- perchlorate de 4-methyl-3-[(4-nitrophenyl)methylene]-1,2-diphenyl-3H-cyclopenta[b]quinolinium
- bis(2-oxo-2-phenylethylure) de 5-[[1-(2-oxo-2-phenylethyl)pyridinium-4-yl]methylene]-5H-indeno[1,2-b]pyridinium,
- trifluoroacetate de 12-(phenylmethylene)-12H-Indolo[2,1-a]isoquinolinium ;
- Sel de 9-(1,3-benzodioxol-5-ylmethylene)-3,9-dihydro-2,3,7-trimethyl-8-phenyl-imidazo[4,5-g]indolizin-6-ium ;
- trifluoroacetate de 12-[(3-hydroxy-4-methoxyphenyl)methylene]-12H-indolo[2,1-a]isoquinolinium ;
- perchlorate de 1,4-dihydro-4-(phenylmethylene)-[1,2,4]Triazino[1,6-b]isoquinolin-11-ium ;
- chlorure de 1-[[4-(dimethylamino)phenyl]methylene]-2,3-dihydro-2-oxo-3,5,7-triphenyl-1H-indolizinium;
- iodure de 7,8-dihydro-10-methyl-8-phenyl-7-(phenylmethylene)-pyrazolo[3',4':4,5]pyrrolo[1,2-a]quinolin-11-ium;
- chlorure de 1-[p-(dimethylamino)benzylidene)-2,3-dimethyl-6-vinyl-1H-indolizinium;
- iodure de 1,9-dihydro-3-methyl-1-phenyl-9-(phenylmethylene)-pyrazolo[3,4-b]indolizin-4-ium;
- perchlorate de 4-methyl-1,2-diphenyl-3-(phenylmethylene)-cyclopenta[b]quinolinium;
- iodure de 1,9-dihydro-9-[(4-hydroxyphenyl)methylene]-3-methyl-1-phenyl-pyrazolo[3,4-b]indolizin-4-ium ;
- iodure de 9-[[4-(dimethylamino)phenyl]methylene]-3,9-dihydro-2,3,7-trimethyl-8-phenyl imidazo[4,5-g]indolizin-6-ium ;
- iodure de 9-[(2,5-dimethoxyphenyl methylene]-3,9-dihydro-2,3,7-trimethyl-8-phenyl-imidazo[4,5-g]indolizin-6-ium;
- iodure de1,9-dihydro-3-methyl-9-[(4-nitrophenyl)methylene]-1-phenyl pyrazolo[3,4-b]indolizin-4-ium.

16. Composition comprenant, dans un milieu cosmétiquement acceptable approprié à la teinture des fibres kératiniues, au moins un colorant direct défini à l'une des revendications précédentes, à l'exception des composés suivants :

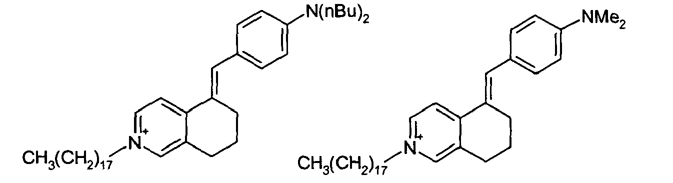

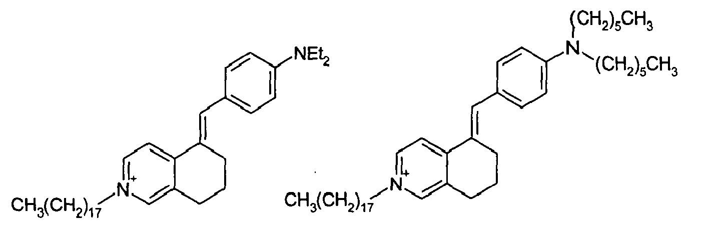

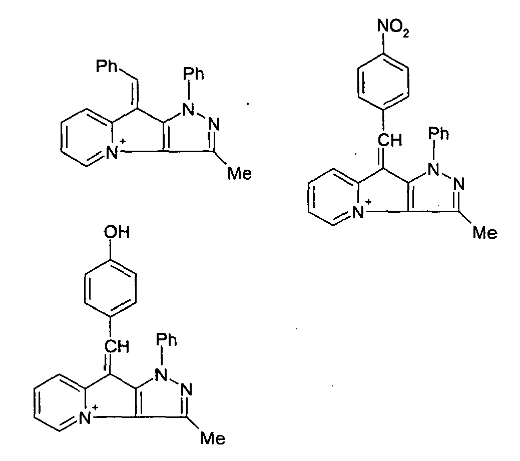

17. Composition selon la revendication 16, **caractérisée en ce que** la teneur en colorant(s) direct(s) est comprise entre 0,01 et 20% en poids par rapport au poids total de la composition.

18. Composition selon la revendication précédente, **caractérisée en ce que** la teneur en colorant(s) direct(s) est comprise entre 0,1 et 5 % en poids, par rapport au poids total de la composition.

19. Composition selon l'une quelconque des revendications 16 à 18, **caractérisée en ce que** le milieu cosmétiquement acceptable est constitué par de l'eau ou par un mélange d'eau et d'au moins un solvant organique.

20. Composition selon l'une quelconque des revendications 9 à 19, **caractérisée en ce qu'**elle comprend en outre au moins un colorant direct additionnel de nature non ionique, cationique ou anionique.

21. Composition selon la revendication précédente, **caractérisée par le fait que** les colorants directs additionnels sont choisis parmi les colorants benzéniques nitrés, les colorants azoïques, les colorants anthraquinoniques, naphtoquinoniques ou benzoquinoniques, les colorants indigoïdes, ou les colorants dérivés du triarylméthane, les colorants naturels, ou leurs mélanges.

22. Composition selon l'une quelconque des revendications 20 ou 21, **caractérisée en ce que** le ou les colorants directs additionnels représentent de 0,0005 à 12 % en poids par rapport au poids total de la composition.

23. Composition selon l'une quelconque des revendications 16 à 22, **caractérisée en ce** q'elle contient au moins un agent tensioactif.

24. Composition selon la revendication précédente, **caractérisée en ce que** l'agent tensioactif est non ionique.

25. Composition selon l'une des revendications 23 ou 24, **caractérisée en ce que** la teneur en tensioactifs représente de 0,01 et 50 % en poids par rapport au poids total de la composition.

26. Composition selon l'une quelconque des revendications 16 à 25, **caractérisée en ce qu'**elle contient au moins un polymère épaississant non associatif.

27. Composition selon la revendication précédente, **caractérisée en ce qu'**elle contient au moins un polymère épaississant non associatif choisi les homopolymères d'acide acrylique réticulés, homopolymères réticulés d'acide 2-acrylamido-2-méthyl-propane sulfonique et leurs copolymères réticulés d'acrylamide, les homopolymères d'acrylate d'ammonium ou les copolymères d'acrylate d'ammonium et d'acrylamide, les gommes de guar non ioniques, les gommes de biopolysaccharides d'origine microbienne, les gommes issues d'exudats végétaux, les hydroxypropyl- ou carboxyméthyl- celluloses ; les pectines et les alginates, seuls ou en mélanges.

28. Composition selon l'une quelconque des revendications 16 à 25, **caractérisée en ce** q'elle contient au moins un polymère épaississant associatif.

29. Composition selon la revendication précédente, **caractérisée en ce qu'**elle contient au moins un polymère épaississant associatif choisi parmi les polyuréthanes associatifs, plus particulièrement cationiques ou non ioniques, les dérivés de cellulose associatifs, plus particulièrement cationiques ou non ioniques, les vinyllactames associatifs, les polyacides insaturés associatifs, les aminoplaste-éthers associatifs, les homopolymères réticulés d'acide 2-acrylamido-2-méthylpropane sulfonique et leurs copolymères réticulés d'acrylamide, les polymères ou copolymères associatifs comprenant au moins un monomère à insaturation éthylénique à groupement sulfonique, seuls ou en mélanges.

30. Composition selon l'une quelconque des revendications 26 à 29, **caractérisée en ce que** la teneur en polymère épaississant associatif ou non représente 0,01 et 10% en poids, plus particulièrement 0,1 à 5% en poids, par rapport au poids de la composition.

31. Composition selon l'une quelconque des revendications 16 à 30, **caractérisée en ce qu'**elle se présente sous la forme d'un shampooing colorant.

32. Composition selon l'une quelconque des revendications 16 à 31, **caractérisée en ce qu'**elle comprend au moins une base d'oxydation éventuellement associée à au moins un coupleur.

33. Composition selon la revendication 32, **caractérisée en ce qu'**elle comprend au moins une base d'oxydation choisie parmi les paraphénylènediamines, les bis-phénylalkylènediamines, les para-aminophénols, les ortho-aminophénols et les bases hétérocycliques ou leurs sels d'addition avec un acide ou avec un agent alcalin.

34. Composition selon la revendication précédente, **caractérisée en ce que** la teneur en base(s) d'oxydation représente de 0,0005 à 12 % en poids par rapport au poids total de la composition.

35. Composition selon l'une quelconque des revendications 32 à 34, **caractérisée en ce qu'**elle comprend au moins un coupleur choisi parmi les métaphénylènediamines, les méta-aminophénols, les métadiphénols et les coupleurs hétérocycliques ou leurs sels d'addition avec un acide ou avec un agent alcalin.

36. Composition selon la revendication précédente, **caractérisée en ce que** la teneur en coupleur(s) représente de 0,0001 à 10 % en poids par rapport au poids total de la composition tinctoriale.

37. Composition selon l'une quelconque des revendications 16 à 30 et 32 à 36, **caractérisée en ce qu'**elle renferme au moins un agent oxydant.

38. Composition selon la revendication précédente, **caractérisée en ce que** l'agent oxydant est choisi parmi le peroxyde d'hydrogène, le peroxyde d'urée, les bromates de métaux alcalins, les persels tels que les perborates et persulfates, et les enzymes.

39. Procédé de traitement de fibres kératiniques, plus particulièrement de fibres kératiniques humaines, **caractérisé en ce que** l'on applique sur lesdites fibres, sèches ou humides, une composition telle que définie selon l'une quelconque des revendications 16 à 38, pendant un temps suffisant pour obtenir la coloration, après quoi on rince, on lave éventuellement au shampooing, on rince à nouveau et on sèche ou on laisse sécher les fibres résultantes.

40. Procédé de traitement de fibres kératiniques, plus particulièrement de fibres kératiniques humaines, **caractérisé en ce que** l'on applique sur lesdites fibres, sèches ou humides, une composition telle que définie selon l'une quelconque des revendications 16 à 38 sans rinçage final.

41. Dispositif à plusieurs compartiments pour la teinture et l'éclaircissement des cheveux, comprenant au moins un compartiment renfermant une composition selon l'une quelconque des revendications 16 à 30 et 32 à 36, et au moins un autre compartiment renfermant une composition renfermant au moins un agent oxydant.

42. Utilisation de la composition selon l'une quelconque des revendications 16 à 31 comme agent colorant et/ou agent d'éclaircissement optique des fibres kératiniques, en particulier des fibres kératiniques humaines.
